# EUROPEAN PATENT SPECIFICATION

(11) **EP 4 110 368 B1**
(45) Date of publication and mention of the grant of the patent: **04.06.2025**
(21) Application number: 21708736.0
(22) Date of filing: 26.02.2021
(51) Int. Cl.: A61K 38/19, A61K 45/06, A61P 1/16, A61K 35/28

(54) **TREATMENT OF LIVER FAILURE**
BEHANDLUNG VON LEBERVERSAGEN
TRAITEMENT DE L'INSUFFISANCE HÉPATIQUE

(30) Priority: 26.02.2020 GB 202002711
(43) Date of publication of application: 04.01.2023
(73) Proprietor: Hepyx Limited, West Midlands WS9 8LZ (GB)
(72) Inventor: ENGELMANN, Cornelius, 15569 Woltersdorf (DE); JALAN, Rajiv, Chislehurst, Kent BR7 5EP (GB); ANDREOLA, Fausto, London Greater London N4 1RT (GB)
(74) Representative: J A Kemp LLP
(86) International application number: PCT/GB2021/050499
(87) International publication number: WO 2021/171036

(56) References cited:
- WO-A1-2012/022939
- WO-A2-2011/119738
- ENGELMANN CORNELIUS ET AL: "Toll-like receptor 4 is a therapeutic target for prevention and treatment of liver failure", JOURNAL OF HEPATOLOGY, ELSEVIER, AMSTERDAM, NL, vol. 73, no. 1, 24 January 2020 (2020-01-24), pages 102 - 112, XP086188903, ISSN: 0168-8278, [retrieved on 20200124], DOI: 10.1016/J.JHEP.2020.01.011
- NORBERTO C. CHAVEZ-TAPIA ET AL: "Granulocyte-colony stimulating factor for acute-on-chronic liver failure: systematic review and meta-analysis", ANNALS OF HEPATOLOGY, vol. 14, no. 5, 1 September 2015 (2015-09-01), MX, pages 631 - 641, XP055738839, ISSN: 1665-2681, DOI: 10.1016/S1665-2681(19)30757-4
- CORNELIUS ENGELMANN ET AL: "Effects of granulocyte-colony stimulating factor (G-CSF) on stem cell mobilization in patients with liver failure", EUROPEAN JOURNAL OF INTERNAL MEDICINE, vol. 36, 1 December 2016 (2016-12-01), NL, pages e37 - e39, XP055738844, ISSN: 0953-6205, DOI: 10.1016/j.ejim.2016.09.006
- SAHA BIPLOB KUMAR ET AL: "Therapeutic implications of granulocyte colony stimulating factor in patients with acute-on-chronic liver failure: increased survival and containment of liver damage", HEPATOLOGY INTERNATIONAL, SPRINGER INDIA, INDIA, vol. 11, no. 6, 24 August 2017 (2017-08-24), pages 540 - 546, XP036373444, ISSN: 1936-0533, [retrieved on 20170824], DOI: 10.1007/S12072-017-9814-1
- ENGELMANN CORNELIUS ET AL: "AS031 - Toll-like receptor 4 inhibition acts synergistically with G-CSF to prevent organ injury and induce liver regeneration in acute-on-chronic liver failure", JOURNAL OF HEPATOLOGY, 1 August 2020 (2020-08-01), pages S28 - S28, XP055797139, Retrieved from the Internet <URL:https://www.sciencedirect.com/science/article/pii/S0168827820306103?via%3Dihub> [retrieved on 20210420], DOI: 10.1016/S0168-8278(20)30610-3

## Description

### Field of the invention

The present invention derives from the unexpected finding that stem cell mobilisers such as G-CSF have a detrimental effect in liver failure, such as acute liver failure (ALF) or acute-on-chronic liver failure (ACLF), by aggravating inflammation and tissue injury but its combination with antagonists of TLR4 acts synergistically by preventing inflammatory driven tissue injury and facilitating stem cell related pro-regenerative properties.

The present invention utilises these findings to provide stem cell mobilisers such as G-CSF and antagonists of TLR4 that may be used in the treatment or prevention of liver failure, such as ALF or ACLF.

### Background to the invention

An acute decompensating event ((AD), bacterial infection, large-volume ascites, GI haemorrhage, or hepatic encephalopathy, alone or in combination) is the most common hospital presentation of cirrhotic liver disease and can be successfully managed in most cases. However, 30% of patients present with or develop rapidly progressive hepatic and/or extra-hepatic organ failure, a condition referred to as acute-on-chronic liver failure (ACLF). About 40 % of these patients progress to multi-organ failure and death. There are currently no specific treatments for ACLF (1).

Acute-on-chronic liver failure (ACLF) affects about 1 in 3 patients hospitalised with a complication of cirrhosis. ACLF is diagnosed by use of the Chronic Liver Failure (CLiF) Consortium criteria, which subclassifies the ACLF severity depending on the number of organ failures into grade 1, 2 and 3. The CLiF Consortium acute-on-chronic liver failure (CLIF-C ACLF) score is superior to conventional scores such as Child-Pugh score or MELD score in predicting death in this cohort (2).

The pathobiology of ACLF is characterised by immunopathological features comprising two opposing mechanisms as the main drivers of organ failure which are active concurrently. The first is systemic and hepatic inflammation triggered by pathogen-associated molecular patterns (PAMPs) and cell death molecules (damage associated molecular patterns (DAMPs)) mediated through the TLR4 receptor pathway. PAMPs derive through translocation of gut-derived bacteria and bacterial products (such as lipopolysaccharide-LPS) to the liver via portal the portal vein, promoting pro-inflammatory responses through the toll-like 4 (TLR4) -dependent canonical inflammasome on immune cells, as well as the TLR4-independent non-canonical inflammasome in hepatocytes (3). Antagonists of TLR4 have been shown to be effective in the treatment of conditions such as ACLF (WO2011GB01227) (4). The second is a prolonged organ injury, immune cell paralysis and failure of regeneration facilitating prolonged organ dysfunction and secondary infections culminating into a vicious cycle leading to multi-organ failure and death. Stem cells and other regulatory immune cells are known to modulate immune activation thereby modulating inflammatory reactions and supporting tissue repair capacities (5). Granulocyte colony stimulating factor (G-CSF) mobilizes stem and immune cells and is perceived to carry immunomodulatory and pro-regenerative properties. Data from a single centre study in India showed an effect of G-CSF for patients with ACLF, improving the survival after 60 days from 30% to 70% (6). Similar data from other Asian centres confirmed the clinical effect, but including again only a small numbers of patients (7, 8). Those results could not be confirmed in more rigorously performed European trials (9, 10). The differences in the results were thought to be due to unbalanced randomisation, poor clinical classification of patients included in the Asian trials and possibly type 1 statistical error. This discrepancy set up the framework for a large multicentre study in Germany, the GRAFT trial, supported by the German Research Foundation (DFG). The GRAFT trial (NCT02669680) recruited 163 patients with ACLF, according to the European-CLIF criteria and performed a planned interim analysis. The data clearly showed that G-CSF has no effect on mortality of patients with ACLF and may be deleterious in a subgroup of patients, e.g. patients with ACLF according to the APASL criteria and alcoholic hepatitis (11).
Engelmann et al., (2020), Journal of Hepatology, 73(1); 102-112 describes Toll-like receptor 4 as a therapeutic target for prevention and treatment of liver failure.
Chavez-Tapia et al., (2015), Annals of Hepatology, 14(5); 631-641 describes granulocyte-colony stimulating factor for the treatment of acute-on-chronic liver failure.
Engelmann et al., (2016), European Journal of Internal Medicine, 36; 37-39 describes effects of granulocyte-colony stimulating factor (G-CSF) on stem cell mobilization in patients with liver failure.
WO2011/119738 A2 describes methods of treating a subject with acute liver injury comprising administering to the subject a therapeutically effective amount of at least one stem cell mobilizer.
WO 2012/022939 A1 describes TLR4 antagonists for use in a method of treating an individual suffering from liver disease presenting with renal or brain dysfunction.
Saha et al., (2017), Hepatology International, 11(6); 540-546 describes therapeutic implications of granulocyte colony stimulating factor in patients with acute-on-chronic liver failure.
Engelmann et al., (2020), Journal of Hepatology, 73; S28 describes that Toll-like receptor 4 inhibition acts synergistically with G-CSF to prevent organ injury and induce liver regeneration in acute-on chronic liver failure.

### Summary of the invention

The present invention is based on the finding that inhibition of TLR4 prevents G-CSF mediated mortality and facilitating G-CSF dependent liver tissue repair capacities. The present invention thus utilises the combination of stem cell mobilisers such as G-CSF and antagonists of Toll like receptor 4 in the treatment and prevention of liver disease, such as ALF or ACLF, and of symptoms and conditions that are associated with liver disease. The synergy consists of an enhanced hepatocyte proliferation and mitigated liver injury.

Accordingly, the invention provides:
A combination of: (a) granulocyte colony stimulating factor (G-CSF), filgrastim, lenograstim, or pegfilgrastim; and (b) an antagonist of TLR4, for use in a method of treating or preventing liver failure in an individual in need thereof.

The invention also provides:
a stem cell mobiliser selected from granulocyte colony stimulating factor (G-CSF), filgrastim, lenograstim, and pegfilgrastim for use in a method of treating or preventing liver failure in an individual in need thereof, wherein the method involves the additional administration of an antagonist of TLR4 to the individual.

The invention provides a combination of: (a) granulocyte colony stimulating factor (G-CSF), filgrastim, lenograstim, or pegfilgrastim; and (b) an antagonist of TLR-4 for use in a method of treating or preventing liver failure such as ALF or ACLF or for treating an individual suffering from liver failure such as ALF or ACLF.

The invention provides a stem cell mobiliser selected from granulocyte colony stimulating factor (G-CSF), filgrastim, lenograstim, and pegfilgrastim for use in a method of treating or preventing liver failure such as ALF or ACLF in an individual or for treating an individual suffering from liver failure such as ALF or ACLF, wherein the method also comprises the administration of an antagonist of TLR4 to the individual.

The invention provides an antagonist of TLR4 for use in a method of treating or preventing liver failure such as ALF or ACLF in an individual or for treating an individual suffering from liver failure such as ALF or ACLF, wherein the method also comprises the administration of a stem cell mobiliser selected from granulocyte colony stimulating factor (G-CSF), filgrastim, lenograstim, and pegfilgrastim to the individual.

In a preferred embodiment, the stem cell mobiliser is G-CSF.

In a preferred embodiment, the liver failure is ACLF.

In particular, the invention provides G-CSF and an antagonist of TLR-4 for use in a method of treating or preventing liver failure such as ALF or ACLF or for treating an individual suffering from liver failure such as ALF or ACLF. The invention provides the combination of G-CSF and an antagonist of TLR-4 for use in a method of treating or preventing liver failure such as ALF or ACLF or for treating an individual suffering from liver failure such as ALF or ACLF.

Similarly, the invention provides G-CSF and an antagonist of TLR-4 for use in a method of treating or preventing liver failure such as ALF or ACLF in an individual in need thereof, said method comprising a step of administering to said individual G-CSF and an antagonist of TLR-4.

Similarly, the invention provides an antagonist of TLR-4 for use in a method of treating or preventing liver failure such as ALF or ACLF in an individual in need thereof, said method comprising a step of administering to said individual an antagonist of TLR-4, wherein G-CSF is also administered to the patient.

The individual to be treated may be suffering from cirrhosis, such as alcoholic cirrhosis. The individual to be treated may be suffering from liver failure. The individual to be treated may be suffering from paracetamol overdose. The individual may be suffering from hepatorenal syndrome (HRS). The individual may be suffering from, or at risk of one or more of the following, when compared to a subject not suffering from liver disease: renal dysfunction; renal failure; HRS; brain dysfunction and brain swelling; increased plasma creatinine; increased plasma ammonia; increased liver enzyme concentrations; increased inflammation, injury or dysfunction in the liver and/or kidney and/or brain and/or blood circulation; liver tissue damage resulting from liver failure; acute liver failure, alcoholic hepatitis, and/or reperfusion injury of the liver. In a preferred embodiment, the individual is suffering from ACLF. In a preferred embodiment, the individual is suffering from ALF. In a preferred embodiment, the individual is suffering from alcoholic hepatitis (AH). In a preferred embodiment, the individual is suffering from nonalcoholic fatty liver disease (NAFLD) or nonalcoholic steatohepatitis (NASH). The individual suffering from AH, NAFLD or NASH may also be suffering from ACLF.

The TLR4 antagonist for use in accordance with the invention may lead to: (a) decreased expression of TLR4 in the liver and/or kidney and/or brain of the individual; and/or (b) decreased levels of TLR4 in the liver and/or kidney and/or brain of the individual; and/or (c) decreased activity of TLR4 in the liver and/or kidney and/or brain of the individual. This would result in reduced inflammation and generation of pro-inflammatory cytokines and reduced dysfunction of organs such as the liver and/or kidneys and/or brain and/or (d) decreased levels of TLR4 in the urine of the individual.

### Brief description of the figures

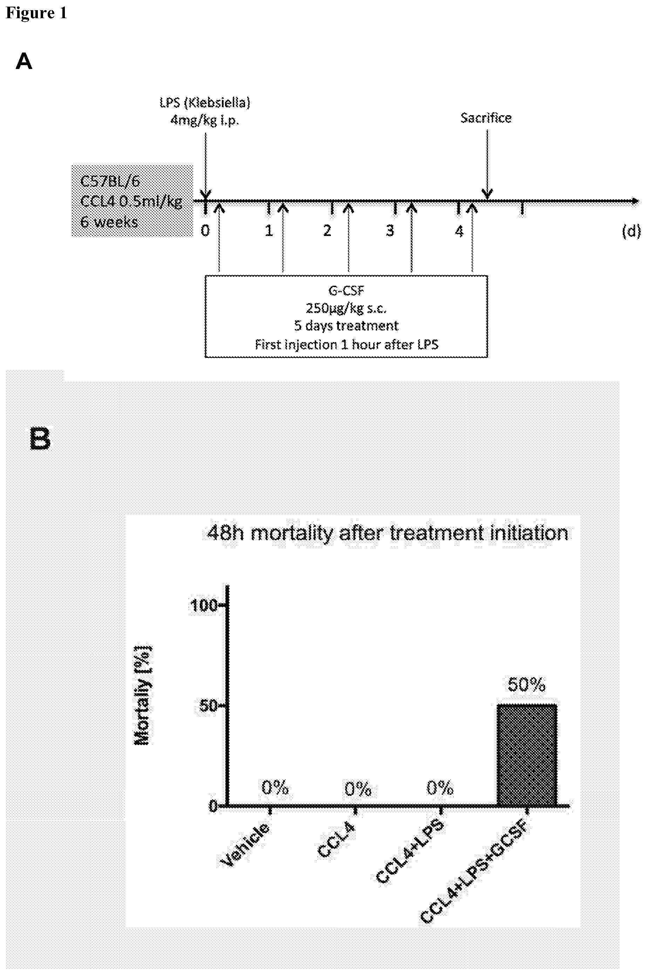

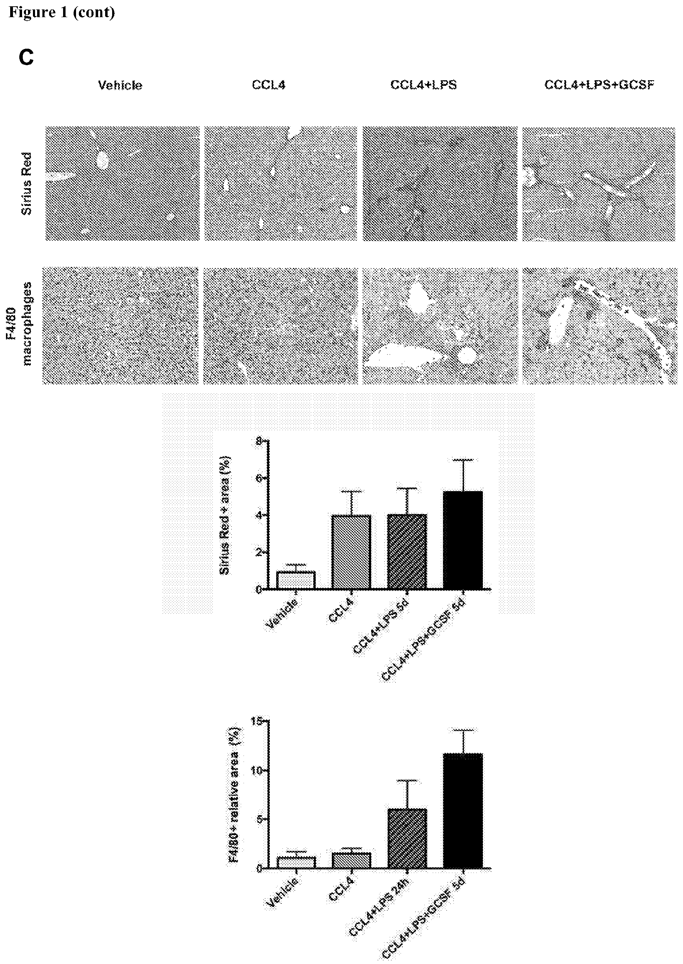

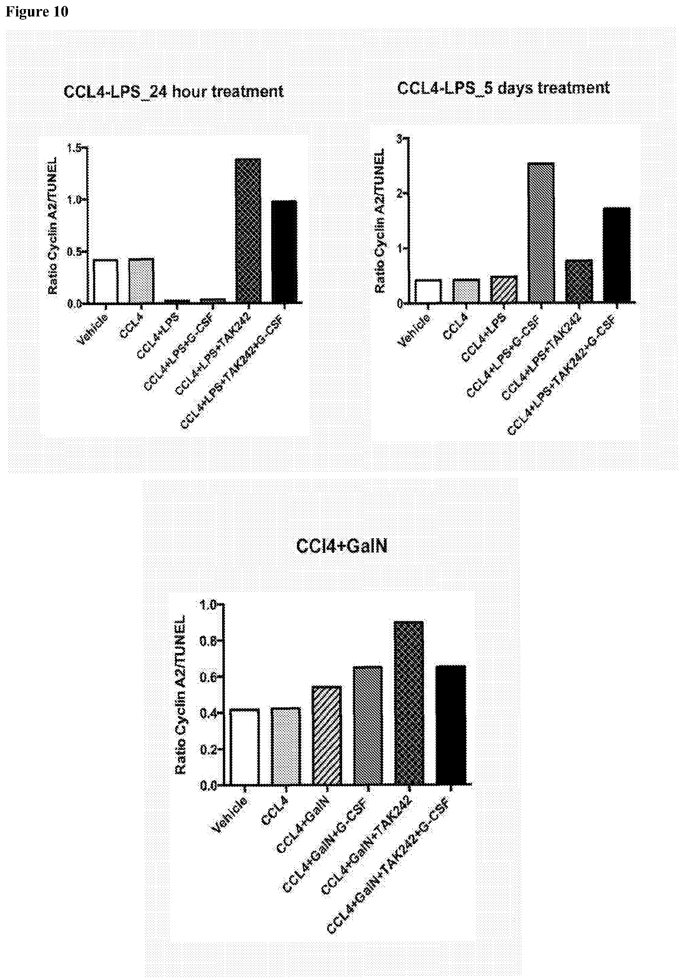

**Figure 1** **- G-CSF increases the mortality of rodents with ACLF**
   **A)** C57B/6 mice were gavaged for 6 weeks with carbontetrachloride (CCL4) (n=6) in order to induce a chronic liver injury. Thereafter injection of LPS (Klebsiella, 4mg/kg, i.p.) served as a second hit to induce organ injury. Mice were treated with recombinant murine G-CSF (250 µg/kg, s.c.) for 5 consecutive days. **B)** In total 50% of all animals treated with G-CSF died within 48 hours after LPS injection whereas all other animals survived the entire treatment episode. **C)** 5-days G-CSF treatment in this ACLF mouse model had a pro-fibrotic effect (Sirius Red) in the liver. Likewise, G-CSF treatment maintained macrophage infiltration (F4/80+) after 5 days. CCL4- Carbontetrachloride; G-CSF - granulocyte colony stimulating factor.
   Data shows that in the CCl4+LPS model G-CSF increased mortality, liver fibrosis and inflammatory cell infiltrates.

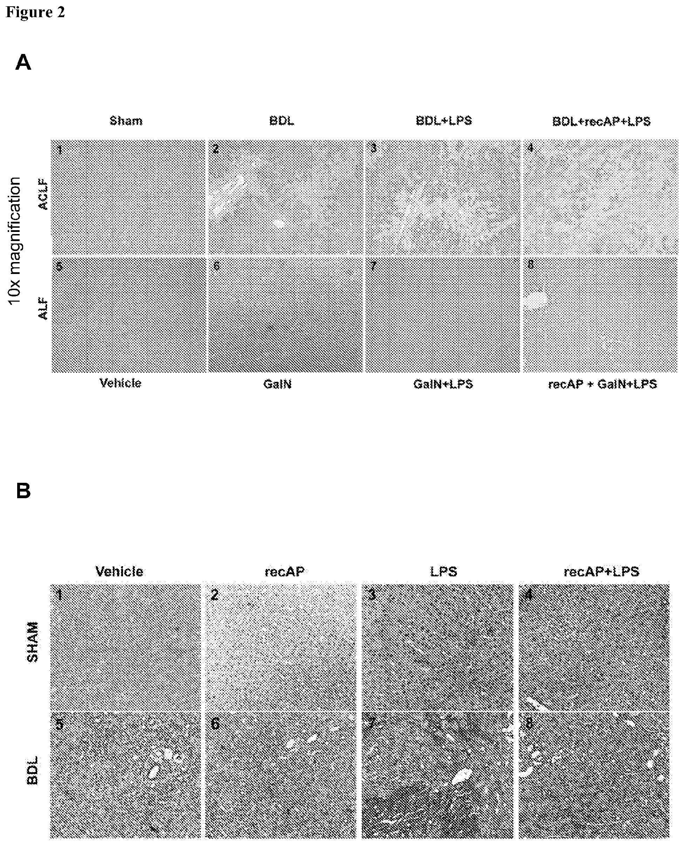

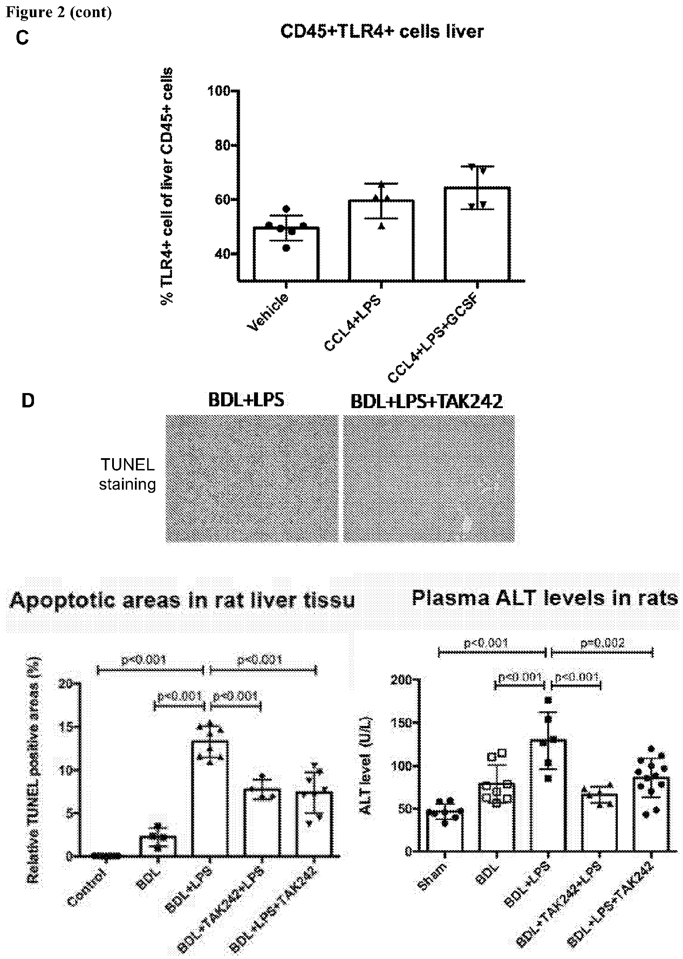

**Figure 2****- Organ sensitisation to endotoxins via TLR4 up-regulation in liver fibrosis. A)** TLR4-expression in liver tissue (immunohistochemistry) from rat of ALF and ACLF models (10x magnification (n = 4 per group)). Liver tissue of control animals showed a low TLR4 expression (A1,A5). Once a chronic injury was induced by BDL there was a clear TLR4 upregulation in hepatocytes whereas regenerative zones did not express TLR4 (A2). LPS injection did not alter this pattern significantly (A3). Pre-treatment with recombinant alkaline phosphatase (recAP) over 4 days (1000U/kg, i.p.), a drug that dephosphorylates and inactivates LPS, reduced TLR4 expression in hepatocytes (A4). In the ALF model, neither GalN alone or with LPS (A6,A7) nor pre-treatment with recAP did alter the TLR4 expression (A8). **B)** TUNEL staining (cell death) of liver tissue confirmed, that chronic liver injury is associated with organ sensitisation to endotoxins, possibly through TLR4 upregulation, as LPS injection after BDL lead to extended areas of cell death (7) whereas the same LPS dose in animals with naive liver showed only low degree cell death (3). The LPS effect could be reverted by recAP pre-treatment (8). C) TLR4+ liver infiltrating CD45+ cells increased after G-CSF treatment. **D)** Using a TLR4-inhibitor (TAK-242) (10mg/kg, i.p ) could provide evidence that organ sensitisation related to chronic liver injury is mediated through TLR4. Injection of TAK-242 before and after LPS injection after BDL significantly reduced liver injury as shown for liver cell death (TUNEL) and ALT levels.

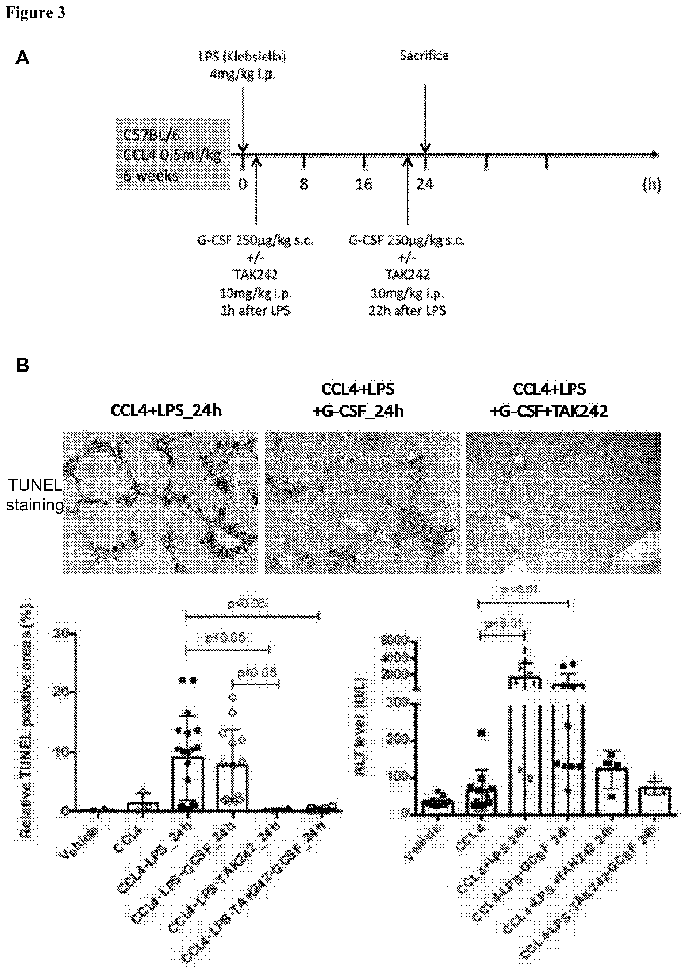

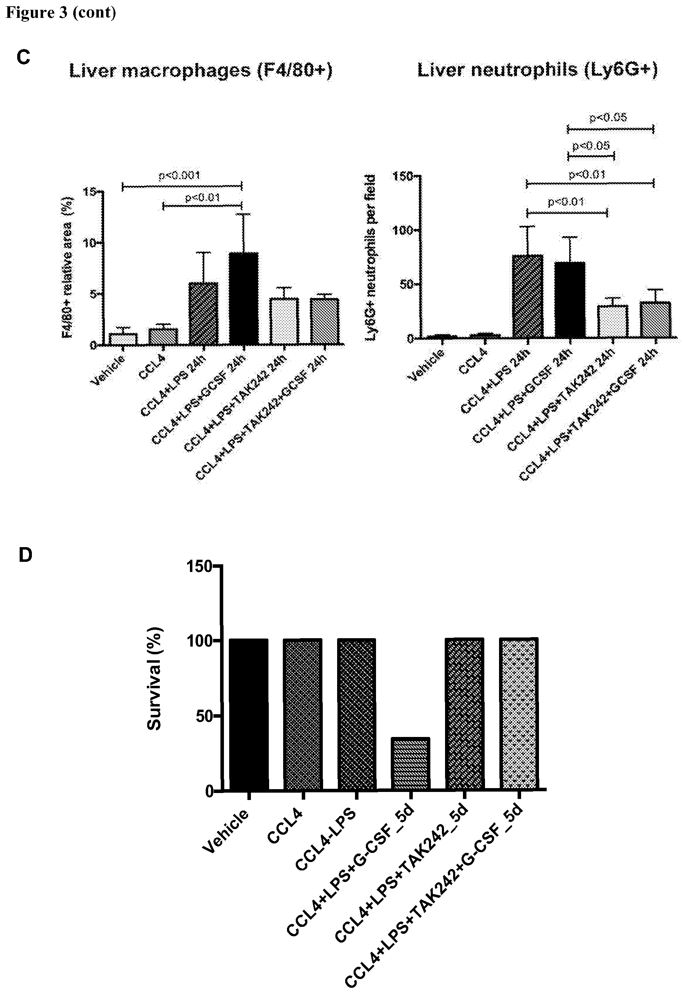

**Figure 3** - **The TLR4 antagonist prevents G-CSF-related mortality and improves liver injury**
   **A)** C57B/6 mice were gavaged for 6 weeks with CCL4 (n=4-10) in order to induce a chronic liver injury. Thereafter injection of LPS (Klebsiella, 4mg/kg, i.p.) served as a second hit to induce organ injury. Mice were treated with recombinant murine G-CSF (250 µg/kg, s.c.) +/- TAK-242 (10mg/kg, i.p) twice for 24h. **B)** LPS injection +/- G-CSF in animals after CCL4 gavage lead to a significant increase of ALT levels and cell death (TUNEL), which was prevented by adding TAK-242. **C)** Liver infiltrating macrophages (F4/80+) and neutrophil granulocytes (Ly6G+) were markedly reduced by adding TAK-242 to G-CSF. **D)** Adding TAK-242 to G-CSF improved animals' survival from 50% to 100% after 48 hours.

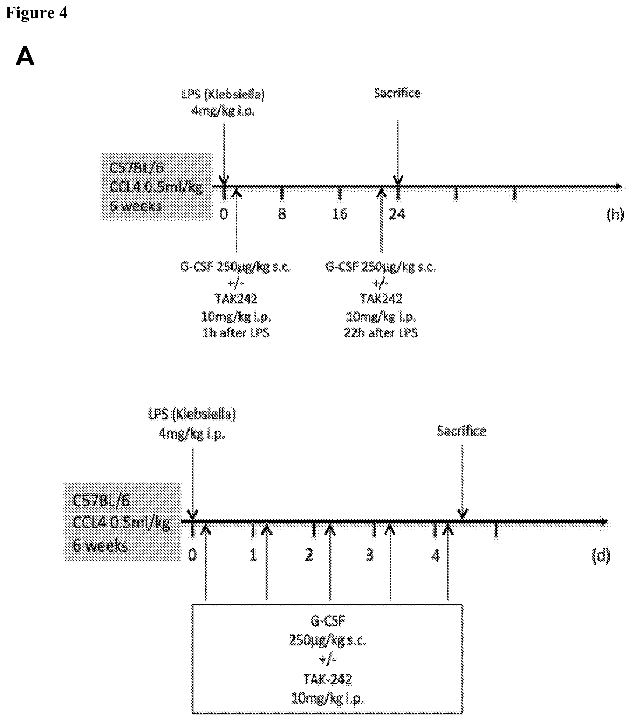

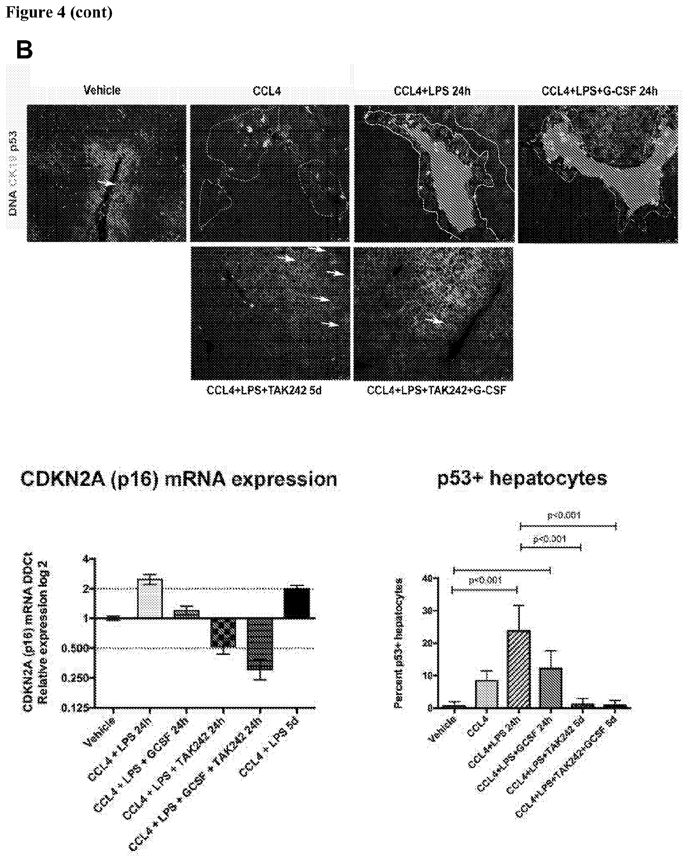

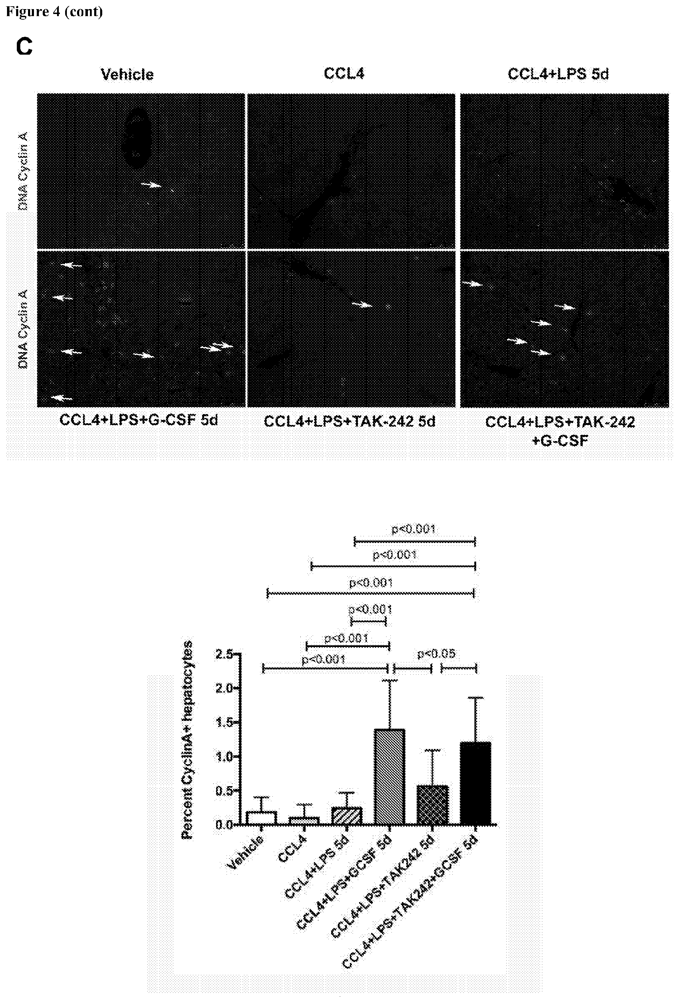

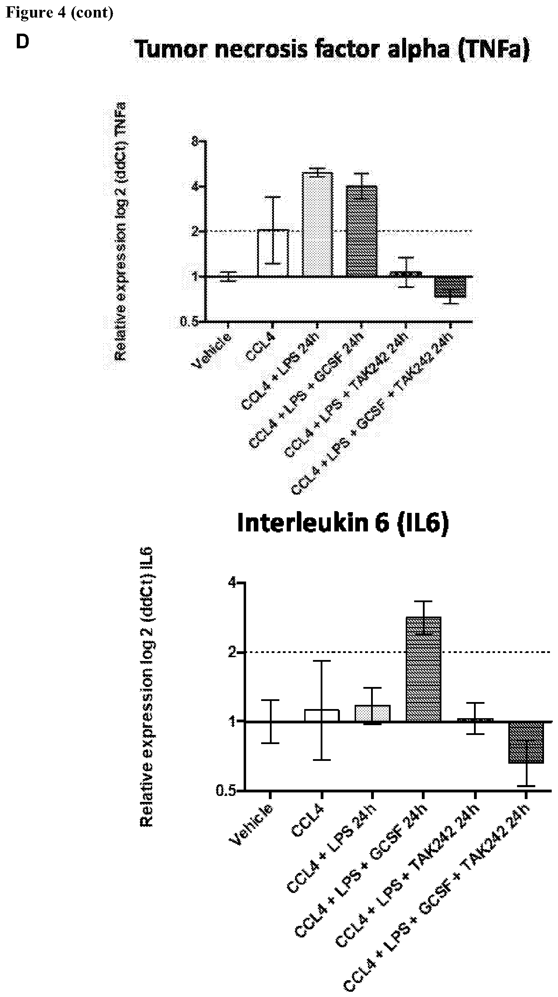

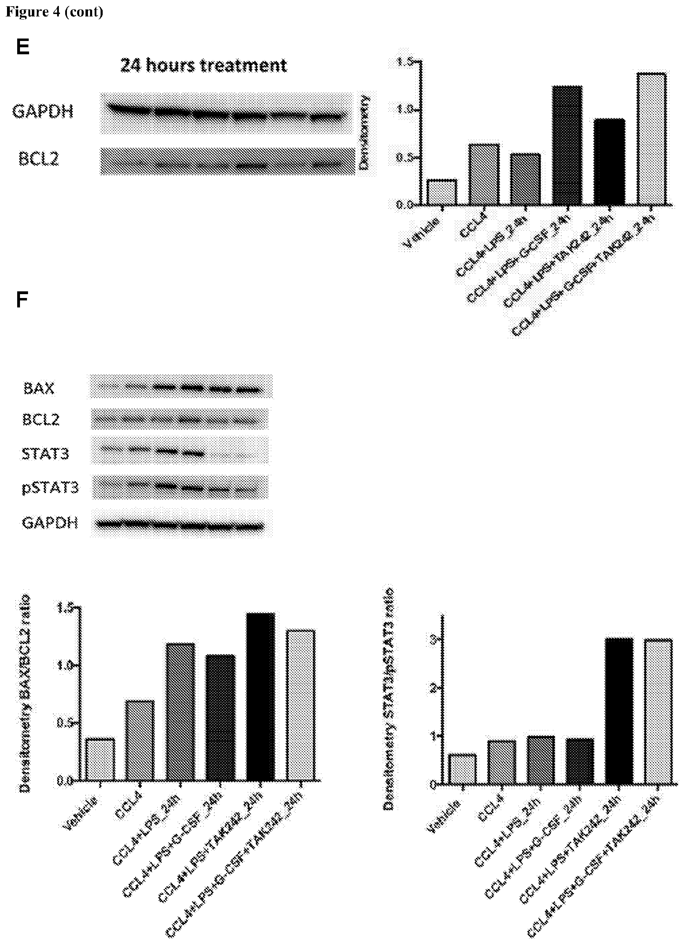

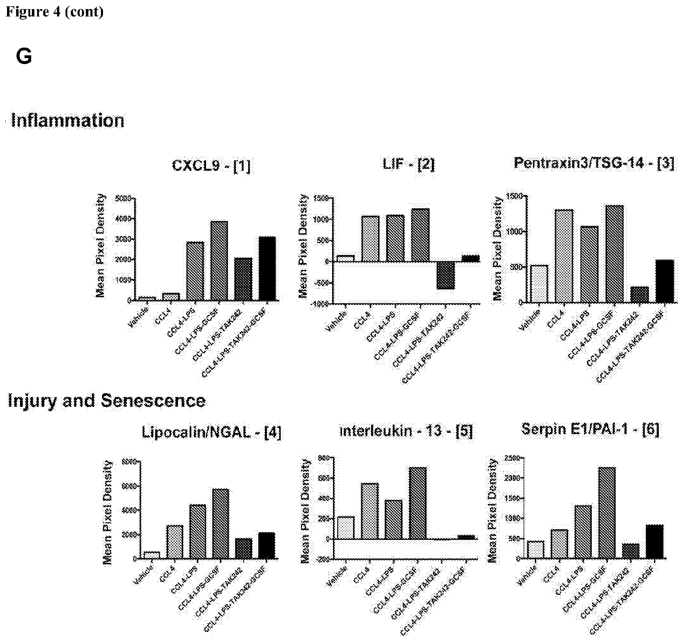

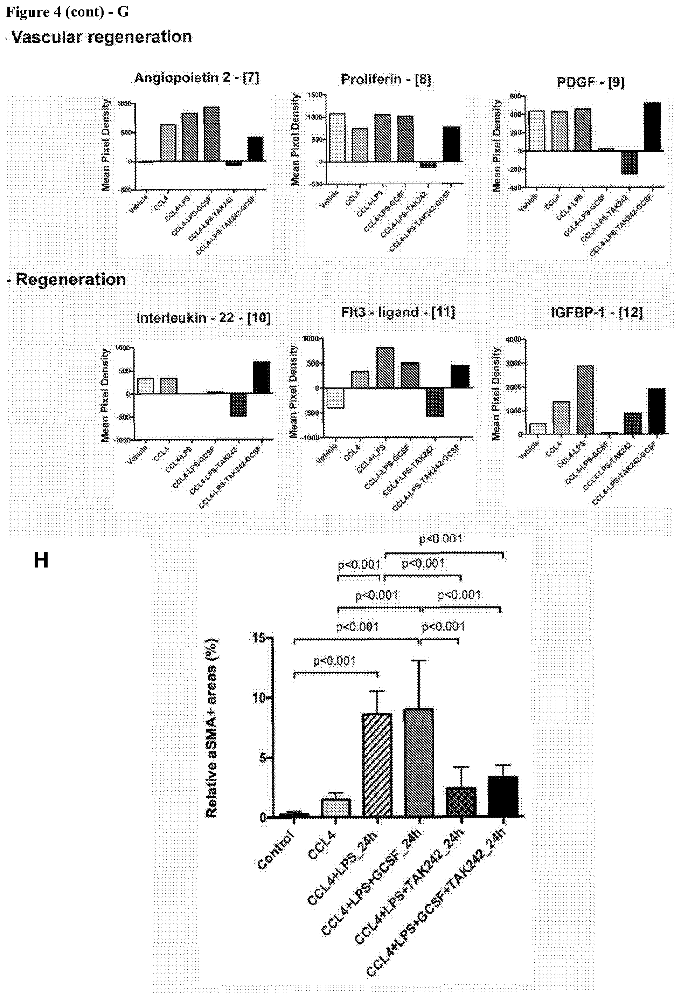

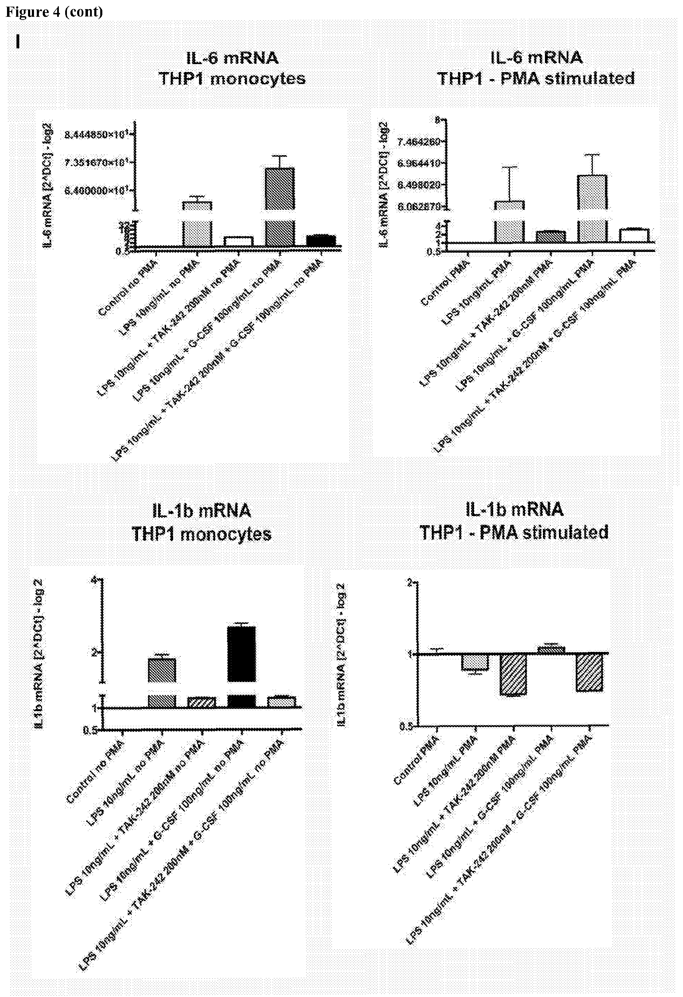

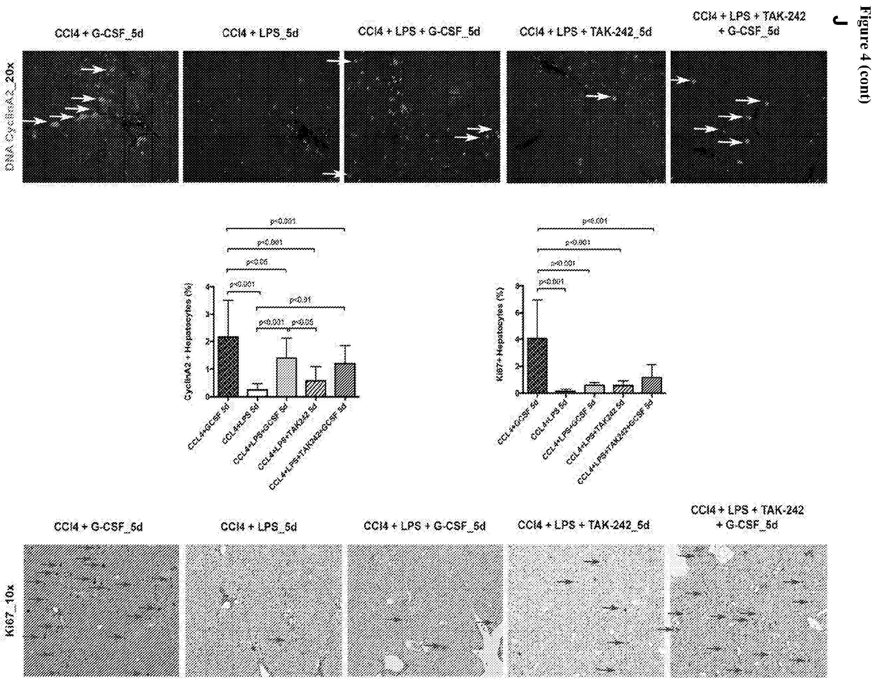

**Figure 4****- Synergistic pro-regenerative effect of G-CSF and TAK-242 in ACLF**
   **A)** C57B/6 mice were gavaged for 6 weeks with CCL4 (n=10) in order to induce a chronic liver injury. Thereafter injection of LPS (Klebsiella, 4mg/kg, i.p.) served as a second hit to induce organ injury. Mice were treated with recombinant murine G-CSF (250 µg/kg, s.c.) +/- TAK-242 (10mg/kg, i.p) either for 24 hours or 5 days. **B)** CDKN2A (p16) which is an important marker of cell cycle arrest (senescence) was upregulated on mRNA level after LPS injection but decreased in a stepwise manner with G-CSF over TAK-242 to the combined therapy. As highlighted by the white shaded areas LPS injection in CCL4 animals led to hepatocellular injury which was surrounded by Trp53 (p53) positive (senescent) hepatocytes. G-CSF alone could not diminish the organ injury (white shaded areas) but reduced numerically the number of p53 positive hepatocytes. After 5 days of treatment with G-CSF+/-TAK-242 the number of senescent hepatocytes plummet significantly. **C)** Cyclin A is a marker of cell cycle progression (proliferation). It majorly regulated the transition from G2 to M phase. In control animals the number of proliferating hepatocytes (white arrow head) was low. After CCL4 gavage and also after subsequent LPS injection there was no increase of Cyclin A positive hepatocytes despite an increasing amount of proliferating immune cells. Treatment with G-CSF over 5 days with or without TAK-242 increased the number of proliferating hepatocytes significantly whereas with TAK-242 single treatment cells were not proliferating. **D)** Hepatic cytokine expression (mRNA) (IL6, TNFa) was increased in ACLF and single treatment with G-CSF but could be reduced by TAK242. Therefore, the combinatory therapy could markedly reduce hepatic inflammation. **E)** BCL2 is the effector molecule of the STAT3 pathway which carries anti-apoptotic and antibacterial properties. The combinatory therapy increased the BCL2 expression in the liver (Western Blot). **F)** The combination therapy of TAK-242 and G-CSF activated the STAT3 pathway with increased expression of anti-apoptotic BCL2 (pooled liver lysates from all animals per group). Image J was used for image quantification and group comparison was performed by one-way ANOVA and post-hoc Tukey's multiple comparison mRNA data is delineated as ddCt value. Western blots were performed with protein lysates pooled from all animals per group. **G)** Liver protein expression of markers of inflammation, cell death and regeneration/senescence (CCl4-LPS 24h treatment model). Liver lysates from 4 randomly selected animals per group were pooled and applied onto one profiler membrane.
   Densitometry was measured by using ImageJ and values are depicted as mean pixel density. After sample pooling no statistical comparison could be applied. **H)** Liver aSMA expression (CCl4-LPS 24 hours treatment model) Liver sections were stained for aSMA (n=4 per group) as a marker of stellate cell activation. LPS increased the aSMA positive area from 1.4% ± 0.6 in CCl4 animals to 8.6% ± 1.9 (p<0.001) and 9% ± 4.1 (p<0.001) when combined with G-CSF. Adding TAK-242 to G-CSF reduced the stellate cell activation significantly (aSMA positive area 3.3% ± 1, p<0.001 compared to CCl4 + LPS + G-CSF). Image J was used for image quantification and group comparison was performed by one-way ANOVA and post-hoc Tukey's multiple comparison. **I)** THP1 response to LPS after G-CSF stimulation. THP1 were PMA activated into a macrophage like phenotype and stimulated with LPS (10ng/mL) ± G-CSF (100ng/mL) (2 batches in 2 replicates). Treatment was performed with TAK-242 200nM in two of the groups. RNA extraction to measure cytokine response was performed 3-hr after LPS incubation. LPS induced an upregulation of IL-6 mRNA expression 60.2-fold in THP1 monocytes and 6.2-fold in macrophages which was enhanced by pre-incubation with G-CSF (monocytes 71-fold, macrophages 6.7-fold). TAK-242 reduced IL-6 mRNA expression compared to unstimulated cells to 5.1-fold in monocytes and 2.8-fold in macrophages resembling biological significance. In monocytes, LPS incubation increased expression of IL-1b 1.8-fold and reduced the expression 0.8-fold in macrophages. G-CSF exaggerated the IL-1b expression 2.5-fold compared to unstimulated cells whereas it remained unchanged in macrophages (1.1-fold compared to unstimulated cells). TAK-242 decreased the IL-1b response in both cell lines (monocytes 1.3-fold and macrophages 0.7-fold compared to unstimulated cells). **J)** Proliferative effect of G-CSF after CCl4. To confirm that G-CSF exhibits pro-proliferative effects in an environment without LPS-driven inflammation, G-CSF was used as a treatment after CCl4 administration. C57B/6 mice were gavaged for 6-weeks with carbon tetrachloride (CCl4) 0.5ml/ml to induce chronic liver injury. G-CSF (250µg/kg, s.c.) was injected without previous LPS administration once daily for 5-days. Results were compared to groups administered LPS. G-CSF treatment enhanced Cyclin A2 expressing hepatocytes from 1.4% ± 0.7 in CCl4 + LPS + G-CSF treated animals to 2.1% ± 1.3 (p<0.05) in CCl4 + G-CSF animals. Ki67 expression as a marker of hepatocyte division increased from 0.6% ± 0.2 after CCl4 + LPS + G-CSF to 4.1% ± 2.9 (p<0.001) after CCl4 + G-CSF. Image J was used for image quantification and group comparison was performed by one-way ANOVA and post-hoc Tukey's multiple comparison.

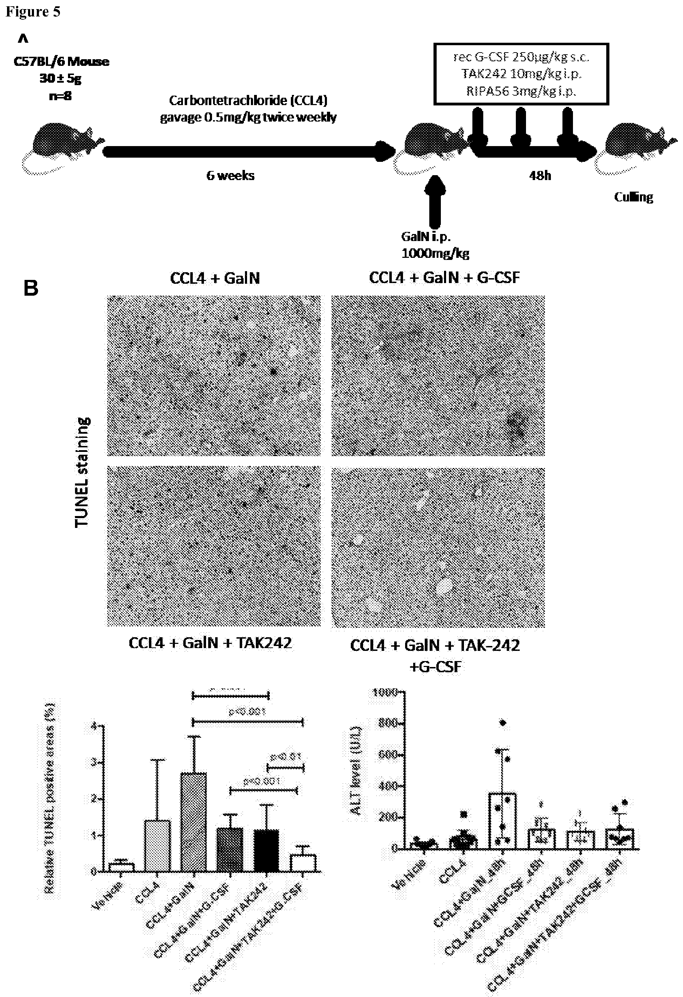

**Figure 5****- Galactosamine (GalN) to induce a non-inflammatory second hit in new developed ACLF mouse model. Effect of G-CSF+TAK-242 on liver injury.**
   **A)** In order to confirm that G-CSF is aggravating the inflammatory response in ACLF and induces a positive treatment effect in a non-inflammatory environment we developed an alternative non-inflammatory ACLF model. C57B/6 mice were gavaged for 6 weeks with CCL4 (n=8) in order to induce a chronic liver injury. Thereafter injection of Galactosamine (GalN) (1000mg/kg, i.p.) served as a primarily non-inflammatory second hit to induce liver injury. Mice were treated with recombinant murine G-CSF (250 µg/kg, s.c.) +/- TAK-242 (10mg/kg, i.p) either for 48 hours. **B)** TUNEL staining and ALT levels revealed that GalN injection was associated with a significant liver injury. The combinatory therapy consisting of G-CSF and TAK-242 abrogated the liver injury and was superior over the individual treatment with either agent to reduce cell death (TUNEL)

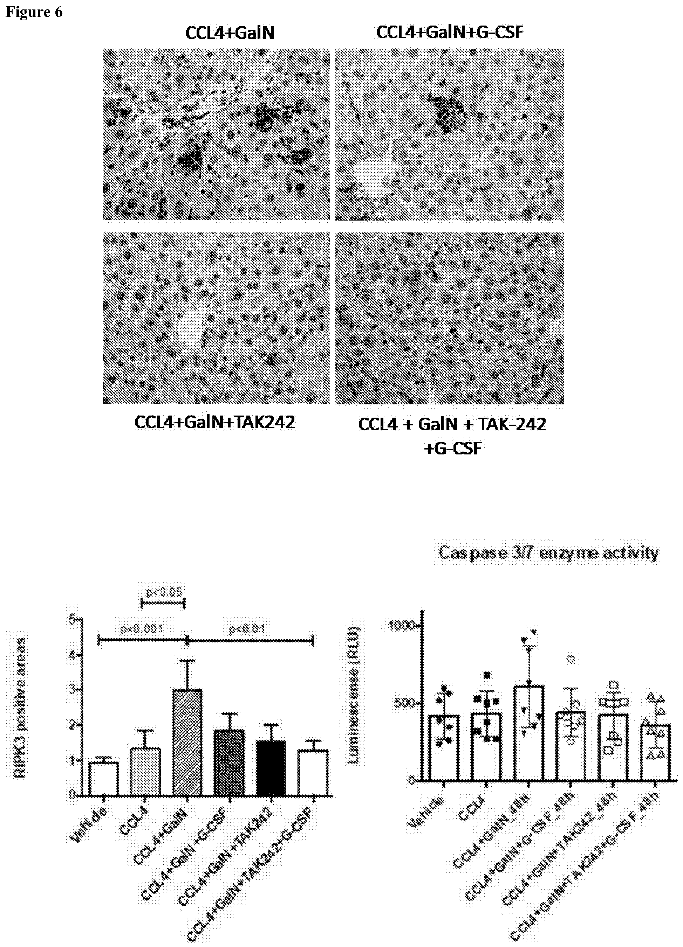

**Figure 6****- Necroptotic liver cell death in liver tissue.**
   The CCl4-GalN model was characterised by necroptotic cell death. After injection of GalN cells were expressing RIPK3, a mediator of necroptosis, whilst caspase 3/7 enzyme activity, a mediator of apoptotic cell death, remained unchanged. Treatment with G-CSF+TAK-242 significantly reduced liver RIPK3 expression.

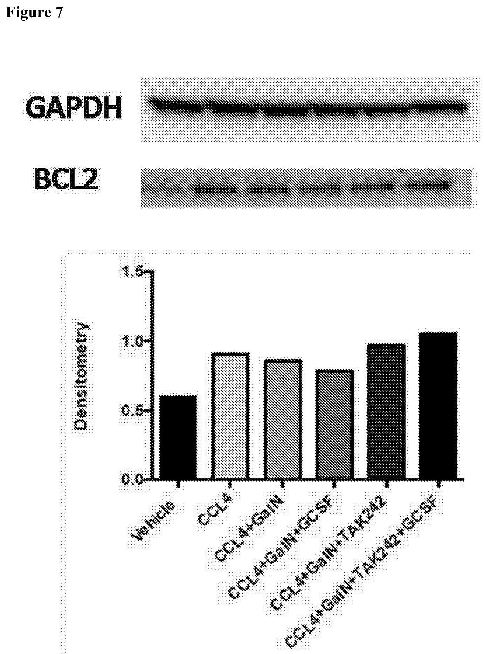

**Figure 7****- BCL2 expression (STAT3 pathway) liver tissue.**
   The combinatory treatment of ACLF with TAK-242 and G-CSF increased liver expression of anti-apoptotic BCL2.
**Figure 8** **- Effect of TAK-242/G-CSF in a non-inflammatory ACLF model.**
   GalN induced a regenerative response with proliferating hepatocytes [Ki67: CCl4 0.1% ± 0.1 vs. CCl4 + GalN 2% ± 1.7, (p<0.01), Cyclin A2: CCl4 0.1% ± 0.2 vs. CCl4 + GalN 1.9% ± 1.6, (p<0.05)] but also hepatocytes in cell cycle arrest [p21: CCl4 0.5% ± 0.2 vs. CCl4 + GalN 6.4% ± 4.7, p<0.01]. G-CSF alone or in combination with TAK-242 reduced cell death and the subsequent regenerative response. TAK-242 alone maintained the amount of proliferating (Cyclin A2, Ki67) and senescent (p21) hepatocytes [(Ki67 CCl4 + GalN + TAK-242 3% ± 2.1 vs. CCl4 + GalN + TAK-242 + G-CSF 1.2% ± 1.4 (p<0.05); Cyclin A2 CCl4 + GalN + TAK-242 1.9% ± 1.6 vs. CCl4 + GalN + TAK-242 + G-CSF 0.3% ± 0.2 (p<0.001; p21 CCl4 + GalN + TAK-242 7.1% ± 4.5 vs. CCl4 + GalN + TAK-242 + G-CSF 1.7% ± 2.3 (p<0.001)].
   Image J was used for image quantification and group comparison was performed by one-way ANOVA and post-hoc Tukey's multiple comparison. mRNA data is delineated as ddCt value and a more the 2 fold change of expression is perceived as biologically significant. Western blots were performed with protein lysates pooled from all animals per group.

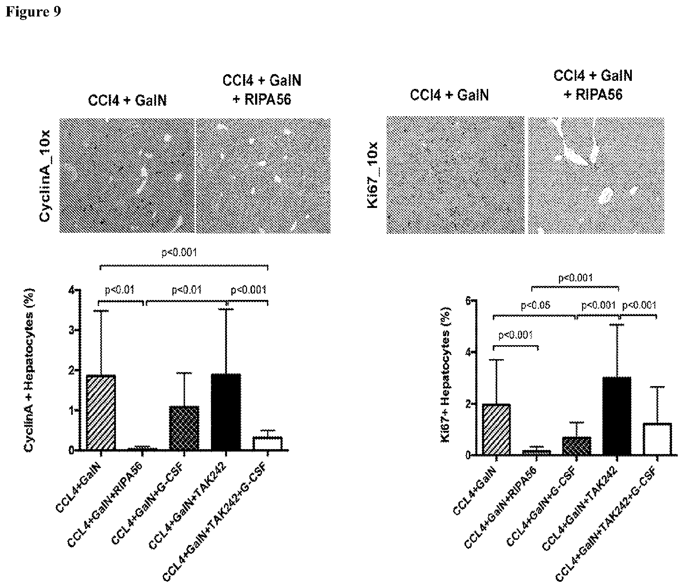

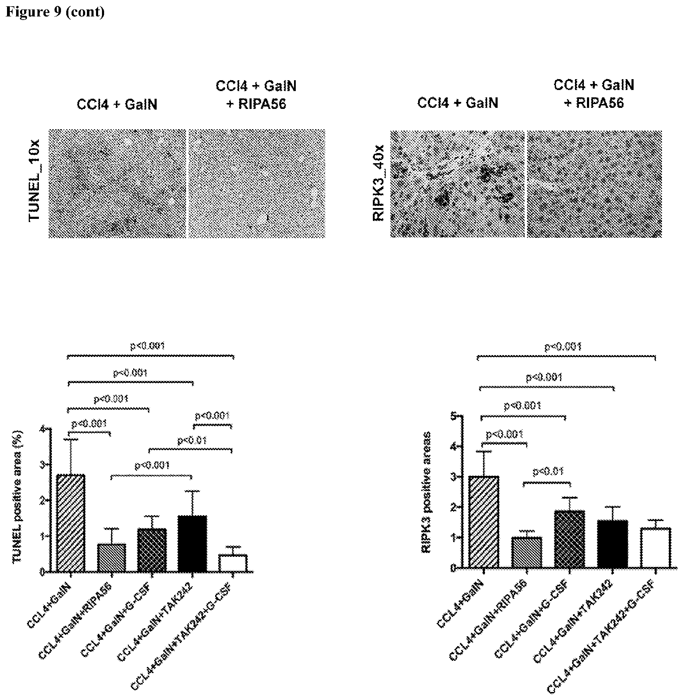

**Figure 9** **- Prevention of liver cell death by RIPA56 prevents proliferative response after CCl4 + GalN.**
   C57B/6 mice were gavaged for 6-weeks with carbon tetrachloride (CCl4) 0.5ml/ml to induce chronic liver injury (n=8 per group). Thereafter, galactosamine (GalN) (1000mg/kg) was administered to induce a non-inflammatory liver injury and treatment with the RIPK1 inhibitor, RIPA56 3mg/kg i.p. was initiated every 12-hrs. RIPA56 treatment reduced GalN-induced cell death (TUNEL p<0.001 (n=4) and RIPK3 expression p<0.001 (n=3) compared to CCl4 + GalN). Diminished liver injury was associated with a decrease in hepatocyte proliferation compared to CCl4 + GalN (Ki67 (n=3): 2% ± 1.7 vs. 0.2% ± 0.2, p<0.001; Cyclin A2 (n=3): 1.9% ± 1.6 vs. 0.03% ± 0.1, p<0.001).
   Image J was used for image quantification and group comparison was performed by one-way ANOVA and post-hoc Tukey's multiple comparison
**Figure 10** **- Ratio between regeneration and liver cell death**
   The ratio between the hepatocyte proliferation marker (Cyclin A2, Ki67) and liver cell death (TUNEL staining) was calculated. An increasing ratio determines an enhanced regenerative response in relation to the degree of liver injury or vice versa. LPS injection, with or without G-CSF led to complete abrogation of hepatocyte proliferation despite high grade of liver injury (CyclinA2/TUNEL ratio: CCl4, 0.42 vs. CCl4 + LPS, 0.02 vs. CCl4 + LPS + G-CSF, 0.04). In contrast, hepatocyte proliferation was preserved after GalN injection (CyclinA2/TUNEL ratio: CCl4 + GalN 0.54). Therapy with TAK-242 enhanced hepatocyte regeneration in both short-term models (LPS 24-hr treatment, GalN 48-hr treatment) (CyclinA2/TUNEL ratio: CCl4 + LPS + TAK-242, 1.4; CCl4 + GalN + TAK-242, 0.9). G-CSF was able to unfold its pro-regenerative properties in the long-term LPS models after 5-days of treatment (CyclinA2/TUNEL ratio: CCl4 + LPS + TAK-242 + G-CSF, 1.7).

### Detailed description of the invention

It is to be understood that different applications of the disclosed methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural references unless the content clearly dictates otherwise. Thus, for example, reference to "an antagonist" includes "antagonists", reference to "an antibody" includes two or more such antibodies, and the like.

The inventors have unexpectedly found that ACLF is can be treated by the use of a combination of: (a) granulocyte colony stimulating factor (G-CSF), filgrastim, lenograstim, or pegfilgrastim and (b) an antagonist of TLR4.

### Stem cells

Stem cells have the ability to differentiate into various cell types in response to appropriate signals. These properties provide stem cells with capabilities for tissue repair, replacement, and regeneration. Accordingly, human stem cells more particularly human embryonic stem cells (hESCs) are of particular interest in medical research. Embryonic stem cells have the ability to differentiate into more cell types than adult stem cells hence have great potential in therapy. Differentiation is triggered by various factors *in vivo,* some of which can be replicated in *in vitro* stem cell cultures. Induced Pluripotent Stem Cells (iPSCs) are a form of stem cell, often used in autologous treatments as they can be created from the tissue of the same patient that will receive the transplant thus avoiding immune rejection. iPSCs obtained in this way do not have the ethical considerations of stem cells derived from embryos. The stem cells may be autologous, and thus derived from the individual to be treated. The stem cells may be heterologous, and thus are not derived from the individual to be treated. The stem cells may be human stem cells. The stem cells may be hepatic stem cells. The stem cells may be human hepatic stem cells.

### Stem cell mobilisers

Stem cells and other regulatory immune cells are known to modulate immune activation thereby modulating inflammatory reactions and supporting tissue repair capacities (5). Stem cell mobilisers are compounds that stimulate stem cells to multiply and migrate from the bone marrow into the circulation. Examples of such compounds are granulocyte colony stimulating factor (G-CSF) and its analogs, such as filgrastim, lenograstim and pegfilgrastim etc, GM-CSF, M-CSF, plerixafor (AMD3100), stem cell factor (SCF), vascular endothelial growth factor (VEGF), erythropoietin and placental growth factor (PGF), CXCL12/CXCR4 modulator, SIP agonists, VCAM/VLA-4 inhibitors, parathyroid hormone, proteasome inhibitors, and Groß, stabilisation of HIF.

### G-CSF

Granulocyte colony stimulating factor (G-CSF) is a glycoprotein that mobilizes stem and immune cells and is perceived to carry immunomodulatory and pro-regenerative properties. It is also known as colony-stimulating factor 3 (CSF 3). G-CSF is produced be a range of different tissues and stimulates the bone marrow to produce stem cells and granulocytes and release them into the bloodstream. G-CSF also acts to stimulate the survival, proliferation, differentiation, and function of neutrophil precursors and mature neutrophils. In the present invention recombinant analogs of G-CSF, or biosimilars of G-CSF, are contemplated to be used instead of G-CSF. The term "G-CSF" is intended to encompass analogs of G-CSF, or biosimilars of G-CSF. In the present invention, analogues of G-CSF include filgrastim, lenograstim and pegfilgrastim.

### TLR4

Toll-like receptor 4 (TLR4) is an important pattern recognition receptor for lipopolysaccharides (LPS) and other gram-negative endotoxins and is expressed on a multitude of non-parenchymal and parenchymal cells, including hepatocytes and hepatic stellate cells. Its activation results in NfKB-mediated inflammatory response.

The present invention relates to the use of a combination of granulocyte colony stimulating factor (G-CSF), filgrastim, lenograstim, or pegfilgrastim and antagonists of TLR4 in the treatment and prevention of liver failure such as acute-on-chronic liver failure (ACLF).

### ALF and ACLF

Acute liver failure (ALF) occurs in individuals with previously normal livers and is due to overwhelming liver injury and usually triggered by a hepatotoxic insult.

Acute-on-chronic liver failure (ACLF) is a distinct clinical entity encompassing an acute deterioration of liver function in patients with cirrhosis, often decompensated cirrhosis, which is usually associated with a precipitating event and results in the failure of one or more organs and high short term mortality. Unregulated inflammation is thought to be a major contributing factor. A characteristic feature of ACLF is its rapid progression, the requirement for multiple organ supports and a high incidence of short and medium term mortality of 40-90%.

The present invention thus derives from the inventors' findings of synergic effect of the combination of antagonists of TLR4 and the stem cell mobiliser G-CSF. The present invention utilises these effects by proposing a combination of granulocyte colony stimulating factor (G-CSF), filgrastim, lenograstim, or pegfilgrastim and antagonists of TLR4 as therapeutic agents for use in the treatment or prevention of liver disease such as ALF or ACLF.

### Antagonists of TLR4

The present invention relates to the antagonism of Toll like receptor 4 (TLR4). An antagonist of TLR4 may be any compound or molecule that inhibits or decreases the activity, function or amount of TLR4. Preferably the antagonist functions in the liver and/or kidney and/or brain of the patient with liver failure. The antagonist may act preferentially in the liver and/or kidney or may act at a number of locations including the liver and/or kidney and/or brain. Preferably the antagonist leads to a decrease in TLR4 activity, function or amount in the organs of an individual to whom the antagonist is administered, such as in one of more of the liver, kidneys, brain, and the heart of the individual. The antagonist may be targeted to the liver, kidney or other organs such as those listed above during administration as discussed further below.

Preferred antagonists are those that decrease the activity or amount of TLR4 by at least 10%, at least 20%, at least 30%, at least 40%, at least 50%, at least 60%, at least 70%, at least 80% or at least 90% compared to the activity or amount of TLR4 seen in the absence of the antagonist.

An antagonist of TLR4 may reduce the activity or amount of TLR4 to an amount or activity that is the same, similar to, or equivalent to, that seen in an individual not suffering from liver disease. For example, as explained herein, the expression of TLR4 is found to be increased in association with a model of cirrhosis. Use of an TLR4 antagonist in accordance with the present invention may lead to a reduction in TLR4 expression in the liver and/or kidneys and/or brain of the individual being treated to a normal level, such as a level that would be seen or would be expected in an individual not suffering from chronic liver disease or cirrhosis.

The antagonist may act specifically to antagonise TLR4. That is, the effect of the antagonist on TLR4 may be greater than any other biological effect of the antagonist. Such an antagonist may be specific to the inhibition of TLR4, that is it may decrease the activity of TLR4, but not other receptors such as other Toll like receptors. Such an antagonist may additionally or alternatively be specific to the expression of TLR4, that is it may decrease the expression of TLR4 but not other receptors such as other Toll like receptors. An antagonist for use in accordance with the present invention may be an antagonist of TLR4 as described herein, that does not act as an antagonist of other Toll like receptors. An antagonist for use in accordance with the present invention may act on TLR4 in preference to other Toll like receptors. For example, an antagonist of TLR4 for use in accordance with the present invention may have one or more of the characteristics of an TLR4 antagonist as described herein, but may not have such characteristics in relation to other Toll like receptors, or may have such characteristics to a lower level in relation to other Toll like receptors when compared to TLR4. For example, an antagonist that decreases the activity of TLR4 may not decrease the activity of other Toll like receptors, or may decrease the activity of other Toll like receptors to a lesser extent, such as a lower percentage decrease, than its effect on TLR4. An antagonist that decreases the expression or amount of TLR4 may not decrease the expression or amount of other Toll like receptors, or may decrease the expression of other Toll like receptors to a lesser extent, such as a lower percentage decrease, than its effect on TLR4. An TLR4 antagonist as described herein may have an effect on other Toll like receptors, such as antagonism of the activity, signalling or expression of one or more other Toll like receptors, that is less than 25%, less than 20%, less than 15%, less than 10%, less than 5%, less than 1%, or less than 0.1% the effect of that antagonist on the activity, signalling or expression of TLR4.

By other Toll like receptors herein is meant any Toll like receptor that is not TLR4. At least 13 groups of Toll like receptor have been identified in mammals. The other Toll like receptor may be any such Toll like receptor that is not TLR4. The other Toll like receptor may be one or more of these Toll like receptors. The other Toll like receptor may be all other Toll like receptors that are not TLR4.

The specificity of the TLR4 antagonist may apply within the whole body of the individual to be treated, that is the actions of the TLR4 antagonist may be specific as discussed above throughout the body of the individual. The specificity of the TLR4 antagonist may apply within particular tissues of the individual, such as the liver, kidneys and/or heart and/or brain. That is, in one embodiment, the TLR4 antagonist may act specifically to antagonise TLR4 as discussed above within the liver and/or kidney and/or other organs of the individual being treated.

The TLR4 antagonist may therefore be a specific antagonist of TLR4 as described above. For example, the TLR4 antagonist may not be an antagonist of other Toll like receptors, or may have no significant effect on the activity or expression of other Toll like receptors.

Any agent capable of inhibiting the activity or function of TLR4 may be suitable for use in the methods of the present invention. Antagonists for use in accordance with the present invention may be direct or indirect antagonists of TLR4.

Direct antagonists are agents whose activity is directly on TLR4. For example, direct antagonists may be agents that act directly on the TLR4 receptor to decrease its activity. A direct antagonist may be an agent that disrupts TLR4 function or that destabilises the TLR4 receptor. A direct antagonist may decrease the amount of TLR4 by destroying or disrupting TLR4 molecules within the patient. A direct antagonist may be an agent that acts on the TLR4 gene, promoter or other gene regulatory regions to decrease expression of the TLR4. A direct antagonist may decrease expression of TLR4 by preventing or reducing expression from the endogenous TLR4 gene.

A TLR4 antagonist may act to disrupt the activity of TLR4. For example, the antagonist may act by preventing activation of TLR4 or y preventing formation of functional complexes comprising TLR4.

Any agent or molecule having the properties described above may be used as an TLR4 antagonist in accordance with the present invention. The test agent may be, or may comprise, for example, a peptide, polypeptide, protein, antibody, polynucleotide, small molecule or other compound that may be designed through rational drug design starting from known antagonists of TLR4.

Examples of TLR4 antagonists or inhibitors that may be used in accordance with the present invention include:
The peptide STM28 as described in Sugiyama et al (European Journal of Pharmacology 594 (2008) 152-156);
Ethyl (6*R*)-6-[*N*-(2-chloro-4-fluorophenyl)sulfamoyl]cyclohex-1-ene-1-carboxylate (TAK-242) which acts by blocking the signaling mediated by the intracellular domain of TLR4, but not the extracellular domain;
Tetrasodium [(2*R*,3*R*,4*R*,5*S*,6*R*)-4-decoxy-5-hydroxy-6-[[(2*R*,3*R*,4*R*,5*S*,6*R*)-4-[(3*R*)-3-methoxydecoxy]-6-(methoxymethyl)-3-[[(*Z*)-octadec-11-enoyl]amino]-5-phosphonatooxyoxan-2-yl]oxymethyl]-3-(3-oxotetradecanoylamino)oxan-2-yl] phosphate (eritoran),which may be provided as E5564. E5564 contains eritoran tetrasodium as an active ingredient. E5564 blocks receptor signal transduction and inhibits the release of the inflammatory cytokines IL-1 and TNF.

NI-0101 is an anti-TLR4 monoclonal antibody that binds to an epitope on TLR4 which interferes with its dimerisation required for intracellular signalling and induction of pro-inflammatory pathways. NI-0101 is a product of NovImmune SA.

OxPAPC (1-palmitoyl-2-arachidonyl-sn-glycero-3-phosphorylcholine), which is an oxidized phospholipid that has been shown to inhibit the signaling induced by bacterial lipopeptide and lipopolysaccharide (LPS).

IAXO compounds such as IAXO-101 (Methyl 6-deoxy-6-N-dimethyl-N-cyclopentylammonium-2, 3-di-O-tetradecyl-α-D-glucopyranoside iodide), IAXO-102 Methyl 6-Deoxy-6-amino-2,3-di-O-tetradecyl-α-D-glucopyranoside, or IAXO-103 (N-(3,4-Bis-tetradecyloxy-benzyl)-N-cyclopentyl-N,N-dimethylammonium iodide)

Compounds that target TLRs such as TLR4 are reviewed in Hennessy et al (2010) Nature Reviews Drug Discovery 9: 293-307.

Preferably the TLR4 antagonist is not LPS.

The TLR4 antagonist may be a molecule that is capable of binding to and preventing or disrupting the activity of TLR4.

Accordingly, one group of TLR4 antagonists for use in accordance with this invention are anti-TLR4 antibodies. Such an antibody may be monoclonal or polyclonal or may be an antigen-binding fragment thereof. For example, an antigen-binding fragment may be or comprise a F(ab)2, Fab or Fv fragment, i.e. a fragment of the "variable" region of the antibody, which comprises the antigen binding site. An antibody or fragment thereof may be a single chain antibody, a chimeric antibody, a CDR grafted antibody or a humanised antibody.

An antibody may be directed to the TLR4 molecule, i.e. it may bind to epitopes present on TLR4 and thus bind selectively and/or specifically to TLR4. An antibody may be directed to another molecule that is involved in the expression and/or activity of TLR4. For example, a polyclonal antibody may be produced which has a broad spectrum effect against one or more epitopes on TLR4 and/or one or more other molecules that are involved in the expression and/or activity of TLR4.

Antibodies can be produced by any suitable method. Means for preparing and characterising antibodies are well known in the art, see for example Harlow and Lane (1988) "Antibodies: A Laboratory Manual", Cold Spring Harbor Laboratory Press, Cold Spring Harbor, NY. For example, an antibody may be produced by raising antibody in a host animal against the whole polypeptide or a fragment thereof, for example an antigenic epitope thereof, herein after the "immunogen".

An antibody, or other compound, "specifically binds" to a molecule when it binds with preferential or high affinity to the molecule for which it is specific but does substantially bind not bind or binds with only low affinity to other molecules. A variety of protocols for competitive binding or immunoradiometric assays to determine the specific binding capability of an antibody are well known in the art (see for example Maddox et al, J. Exp. Med. 158, 1211-1226, 1993). Such immunoassays typically involve the formation of complexes between the specific protein and its antibody and the measurement of complex formation.

The TLR4 antagonist may be an antisense oligonucleotide, such as an antisense oligonucleotide against the gene encoding a TLR4 protein. The term "antisense oligonucleotide" as used herein means a nucleotide sequence that is complementary to the mRNA for a desired gene. Such an antisense oligonucleotide may selectively hybridise with the desired gene. In the context of the present invention, the desired gene may be the gene encoding TLR4.

The TLR4 antagonist may modulate expression of the TLR4 gene. For example, the TLR4 antagonist may be a short interfering nucleic acid (siRNA) molecule, double stranded RNA (dsRNA), micro RNA, deoxyribose nucleic acid interference (DNAi) or short hairpin RNA (shRNA) molecule.

The term "selectively hybridise" as used herein refers to the ability of a nucleic acid to bind detectably and specifically to a second nucleic acid. Oligonucleotides selectively hybridise to target nucleic acid strands under hybridisation and wash conditions that minimise appreciable amounts of detectable binding to non-specific nucleic acids. High stringency conditions can be used to achieve selective hybridisation conditions as known in the art. Typically, hybridisation and washing conditions are performed at high stringency according to conventional hybridisation procedures. Washing conditions are typically 1-3xSSC, 0.1-1% SDS, 50-70°C. with a change of wash solution after about 5-30 minutes.

The TLR4 antagonist may be a nucleic acid molecule such as an antisense molecule or an aptamer. The nucleic acid molecule may bind a specific target molecule.

Aptamers can be engineered completely *in vitro,* are readily produced by chemical synthesis, possess desirable storage properties, and elicit little or no immunogenicity in therapeutic applications. These characteristics make them particularly useful in pharmaceutical and therapeutic utilities.

The terms "nucleic acid molecule" and "polynucleotide" are used interchangeably herein and refer to a polymeric form of nucleotides of any length, either deoxyribonucleotides or ribonucleotides, or analogs thereof. A nucleic acid may comprise conventional bases, sugar residues and inter- nucleotide linkages, but may also comprise modified bases, modified sugar residues or modified linkages. A nucleic acid molecule may be single stranded or double stranded.

In general, aptamers may comprise oligonucleotides that are at least 5, at least 10 or at least 15 nucleotides in length. Aptamers may comprise sequences that are up to 40, up to 60 or up to 100 or more nucleotides in length. For example, aptamers may be from 5 to 100 nucleotides, from 10 to 40 nucleotides, or from 15 to 40 nucleotides in length. Where possible, aptamers of shorter length are preferred as these will often lead to less interference by other molecules or materials.

Aptamers may be generated using routine methods such as the Systematic Evolution of Ligands by EXonential enrichment (SELEX) procedure. SELEX is a method for the *in vitro* evolution of nucleic acid molecules with highly specific binding to target molecules. It is described in, for example, US 5,654,151, US 5,503,978, US 5,567,588 and WO 96/38579. The SELEX method involves the selection of nucleic acid aptamers and in particular single stranded nucleic acids capable of binding to a desired target, from a collection of oligonucleotides. A collection of single-stranded nucleic acids (e.g., DNA, RNA, or variants thereof) is contacted with a target, under conditions favourable for binding, those nucleic acids which are bound to targets in the mixture are separated from those which do not bind, the nucleic acid-target complexes are dissociated, those nucleic acids which had bound to the target are amplified to yield a collection or library which is enriched in nucleic acids having the desired binding activity, and then this series of steps is repeated as necessary to produce a library of nucleic acids (aptamers) having specific binding affinity for the relevant target.

Any of the antagonists described herein may therefore be used to antagonise TLR4, i.e. to decrease the amount of TLR4 that is present, and/or the activity or the function of the TLR4. Preferably these antagonising effects take place in the liver and/or kidney and/or brain.

An antagonist of TLR4 may be an agent that decreases the production of endogenous TLR4. For example, the agent may act within the cells of the subject to inhibit or prevent the expression of TLR4. Such an agent may be a transcription factor or enhancer that acts on the TLR4 gene to inhibit or prevent gene expression.

Preferably the antagonist of TLR4 is an agent capable of reducing injury and/or organ dysfunction caused by administration of a hepatotoxin, such as acetaminophen. For example, this ability may be tested in a suitable animal model, such as a non-human animal (e.g. a mouse or rat), that is treated with such a hepatotoxin. The effects of the potential TLR4 antagonist on such an animal may be assessed. The TLR4 antagonist may be administered prior to, at the same time as, or after, administration of the hepatotoxin to the animal. The effects of the hepatotoxin in the presence of the antagonist may be compared to the effects of the hepatotoxin in the absence of the TLR4 antagonist, for example in a vehicle-treated animal. A suitable TLR4 antagonist for use in accordance with the present invention may reduce injury or organ dysfunction in the animal compared to that seen in the absence of the TLR4 antagonist. This reduced injury or dysfunction may be characterised using any of the criteria discussed further herein, such as a reduction in liver enzymes, a reduction in plasma creatinine and/or ammonia levels, an alteration in inflammatory modulator levels such as levels of NF6B or TNFalpha, a reduction in the level of interleukin 1a in the liver, a decrease in brain water, a decrease in tissue damage in the organ, or other characteristics of injury or organ dysfunction that would be expected to result from treatment with a hepatotoxin. The organ may be, for example, the liver, the kidney, the heart and/or the brain. A suitable TLR4 antagonist would be expected to have such improved effects compared with the effects that are seen with administration the hepatotoxin in the absence of the TLR4 antagonist.

### Pharmaceutical formulations

A suitable TLR4 antagonist as described herein is typically formulated for administration with a pharmaceutically acceptable carrier or diluent. The antagonist may thus be formulated as a medicament with a standard pharmaceutically acceptable carrier(s) and/or excipient(s) as is routine in the pharmaceutical art. The exact nature of the formulation will depend upon several factors including the desired route of administration. Typically, the antagonist may be formulated for oral, intravenous, intragastric, intravascular or intraperitoneal administration.

If a stem cell is to be administered, it is typically formulated for administration with a pharmaceutically acceptable carrier or diluent. Formulation of cells with standard pharmaceutically acceptable carriers and/or excipients may be carried out using routine methods in the pharmaceutical art. The exact nature of a formulation will depend upon several factors including the cells to be administered and the desired route of administration. Suitable types of formulation are fully described in Remington's Pharmaceutical Sciences, 19th Edition, Mack Publishing Company, Eastern Pennsylvania, USA. Compositions may be prepared together with a physiologically acceptable carrier or diluent. Typically, such compositions are prepared as liquid suspensions of cells. The cells may be mixed with an excipient which is pharmaceutically acceptable and compatible with the active ingredient. Suitable excipients are, for example, water, saline, dextrose, glycerol, of the like and combinations thereof.

In addition, if desired, the pharmaceutical compositions may contain minor amounts of auxiliary substances such as wetting or emulsifying agents, pH buffering agents, and/or adjuvants which enhance effectiveness.

The stem cell mobiliser such as G-CSF is typically formulated for administration with a pharmaceutically acceptable carrier or diluent. The stem cell mobiliser such as G-CSF may thus be formulated as a medicament with a standard pharmaceutically acceptable carrier(s) and/or excipient(s) as is routine in the pharmaceutical art. The exact nature of the formulation will depend upon several factors including the desired route of administration. Typically, the stem cell mobiliser such as G-CSF may be formulated for oral, intravenous, intragastric, intravascular or intraperitoneal administration.

The stem cell mobiliser such as G-CSF and the antagonist of TLR4 may be present in a single formulation as described herein. The stem cell mobiliser such as G-CSF and the antagonist of TLR4 may be present in a single combination, composition or pharmaceutical composition.

The stem cell mobiliser such as G-CSF and the TLR4 antagonist may be formulated for simultaneous, subsequent or sequential delivery. The TLR4 antagonist may be administered before, at the same time, or after, the stem cell mobiliser such as G-CSF. The stem cell mobiliser such as G-CSF can be administered before, at the same time, or after, the TLR4 antagonist. The pharmaceutical composition may comprise G-CSF and an antagonist of TLR4.

The stem cell mobiliser such as G-CSF and the antagonist of TLR4 may be present in a single formulation as described herein. The stem cell mobiliser such as G-CSF and the antagonist of TLR4 may be present in a single combination, composition or pharmaceutical composition.

The pharmaceutical carrier or diluent may be, for example, an isotonic solution such as physiological saline. Solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, gum arabic, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film-coating processes.

Liquid dispersions for oral administration may be syrups, emulsions or suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with ornithine and at least one of phenylacetate and phenylbutyrate, a pharmaceutically acceptable carrier, e.g. sterile water, olive oil, ethyl oleate, glycols, e.g. propylene glycol, and if desired, a suitable amount of lidocaine hydrochloride.

Where the TLR4 antagonist to be administered is a nucleic acid molecule, for example where the antagonist is in the form of an expression vector, certain facilitators of nucleic acid uptake and/or expression ("transfection facilitating agents") can also be included in the compositions, for example, facilitators such as bupivacaine, cardiotoxin and sucrose, and transfection facilitating vehicles such as liposomal or lipid preparations that are routinely used to deliver nucleic acid molecules.

A pharmaceutical formulation may further comprise one or more additional therapeutic agents. For example, the formulation may comprise one or more TLR4 antagonists as defined herein and a stem cell mobiliser such as G-CSF. The formulation may comprise more than one stem cell mobiliser. The formulation may comprise one or more TLR4 antagonists as described here and also one or more additional therapeutic agents. Preferably the additional therapeutic agent(s) are agents which will assist in the treatment or prophylaxis of the individual to be treated. For example, one or more agents that are effective at treating liver disease may be administered as part of a formulation as described herein. One or more agents that are effective at treating an underlying liver condition or symptom thereof in the patient may be administered as part of a formulation as described herein.

The formulation may comprise one or more TLR4 antagonists as described here and also one or more additional therapeutic agents. Preferably the additional therapeutic agent(s) are agents which will assist in the treatment or prophylaxis of the individual to be treated. For example, one or more agents that are effective at treating liver disease may be administered as part of a formulation as described herein. One or more agents that are effective at treating an underlying liver condition or symptom thereof in the patient may be administered as part of a formulation as described herein.

### Treatment

The present invention provides methods for the treatment of individuals having liver failure such as ACLF, particularly for the treatment or prevention of symptoms and conditions associated with or resulting from liver failure such as ALF or ACLF. Accordingly, the invention provides a combination of (a) granulocyte colony stimulating factor (G-CSF), filgrastim, lenograstim, or pegfilgrastim and (b) an antagonist of TLR4 for use in a method of treating an individual having liver failure such as ALF or ACLF. Also described is the use of a stem cell mobiliser such as G-CSF and an antagonist of TLR4 in the manufacture of a medicament for use in the treatment of an individual having liver failure such as ALF or ACLF.

Accordingly, the invention a combination of: (a) granulocyte colony stimulating factor (G-CSF), filgrastim, lenograstim, or pegfilgrastim and (b) an antagonist of TLR4 for use in a method of treating an individual having liver failure such as ALF or ACLF. Also described is the use of a stem cell mobiliser such as G-CSF and an antagonist of TLR4 in the manufacture of a medicament for use in the treatment of an individual having liver failure such as ALF or ACLF.

The G-CSF and an antagonist of TLR4 may be provided in a formulation as described herein. G-CSF and an antagonist of TLR4 as described herein is thus administered to a subject in order to treat or prevent liver failure such as ALF or ACLF, or particular symptoms or conditions associated with liver failure such as ALF or ACLF in the subject. G-CSF and an antagonist of TLR4 as described herein can thus be administered to improve the condition of a subject, for example a subject suffering from liver failure such as ALF or ACLF. G-CSF and an antagonist of TLR4 as described herein may be administered to alleviate the symptoms of a subject, for example the symptoms associated with liver failure such as ALF or ACLF.

G-CSF and an antagonist of TLR4 as described herein may be administered to combat or delay the onset of liver failure such as ALF or ACLF or any symptom associated therewith. The invention can therefore prevent the medical consequences of liver failure such as ALF or ACLF. Use of G-CSF and an antagonist of TLR4 as described herein may thus extend the life of a patient with liver failure such as ALF or ACLF.

The treatment of liver failure such as ALF or ACLF, refers to the treatment of an individual having or at risk of having liver failure such as ALF or ACLF. The individual may also be suffering from chronic liver disease such as cirrhosis or alcoholic cirrhosis. The patient may be suffering from liver disease or cirrhosis associated with or caused by an infection such as a hepatitis virus infection such as hepatitis C virus infection. The patient may also be suffering from liver disease or cirrhosis associated with or caused by treatment with a hepatotoxin such as acetaminophen (paracetamol). The patient may also be suffering from AH, NAFLD or NASH. The methods described herein may be used in the treatment of any such disease.

The individual may be suffering from one or more symptoms or conditions caused by or associated with liver failure such as ALF or ACLF. Any one or more of these conditions or symptoms may be treated in accordance with the present invention. For example, the individual may be suffering from, or at risk of, one or more of the following as a result of their liver failure such as ACLF: renal dysfunction; renal failure; HRS; increased plasma creatinine; brain dysfunction and brain swelling increased plasma ammonia; increased liver enzyme concentrations (such as increased concentrations of ALT and/or AST in the liver); increased inflammation, injury and/or dysfunction in the liver and/or kidney and/or brain and/or blood circulation; liver tissue damage resulting from liver failure, such as resulting from acetaminophen (APAP) toxicity. The individual may be suffering from, or at risk of, acute liver failure, alcoholic hepatitis and/or reperfusion injury of the liver. Those conditions may result from the ALF and/or ACLF of the individual. The methods and uses described herein may be of utility in the treatment or prevention of any one or more of these symptoms or conditions, particularly, in an individual suffering from liver failure such as ACLF.

In particular, the methods described herein may be used in the treatment of a patient having liver failure such as ALF or ACLF. For example, the patient may have or be at risk or renal failure. The liver failure such as ALF or ACLF may result from an infection and/or inflammation. The liver failure such as ACLF may result from exposure to a hepatotoxin such as acetaminophen (paracetamol) such as exposure to a high level of the hepatotoxin, such as an overdose with paracetamol. The methods described herein may be used to treat or prevent any of these conditions or symptoms.

As described herein, the antagonist of TLR4 may lead to decreased expression and/or decreased levels of TLR4 in the liver and/or kidney of the subject. For example, the antagonist may be an agent that inhibits transcription of TLR4 in cells of the subject.

As described herein, the antagonist of TLR4 may lead to decreased activity of TLR4 in the liver and/or kidney of the individual.

The subject is treated with a combination of: (a) granulocyte colony stimulating factor (G-CSF), filgrastim, lenograstim, or pegfilgrastim and (b) an antagonist of TLR4 as described herein. In a preferred embodiment the subject is treated with G-CSF and an antagonist of TLR4 as described herein. As described above, the stem cell mobiliser such as G-CSF and an antagonist of TLR4 may each be administered alone, subsequently to each other, sequentially to each other, and/or in the form of a pharmaceutical formulation. The formulation may comprise may comprise one or more additional therapeutic or prophylactic agents.

Two or more different TLR4 antagonists as described herein may be used in combination in addition the use of a stem cell to treat a subject. The two or more antagonists may be administered together, in a single formulation, at the same time, in two or more separate formulations, or separately or sequentially as part of a combined administration regimen.

G-CSF and an antagonist of TLR4 may be administered in combination with another agents known to be useful in the treatment or prevention of liver failure such as ALF or ACLF. G-CSF and an antagonist of TLR4 and the other agents may be administered together, in a single formulation, at the same time, in two or more separate formulations, or separately or sequentially as part of a combined administration regimen.

A pharmaceutical composition or formulation of the invention may be administered by any suitable route. Preferably it is administered by oral, intravenous, intragastric, intraperitoneal or intravascular routes. The antagonist or formulation may be administered directly to the liver of the subject.

The pharmaceutical composition or formulation is administered in a therapeutically effective amount. A suitable dose of the pharmaceutical composition or formulation of the invention can be determined according to various parameters such as the age, weight and condition of the subject to be treated; the type and severity of the liver disease; the route of administration; and the required regimen. A suitable dose can be determined for an individual antagonist. For example, for some pharmaceutical compositions or formulations a typical dose may be in the order of from 0.1 mg/kg/day to 30 g/kg/day. A physician will be able to determine the required dosage of antagonist and for any particular subject.

The present invention is broadly applicable to therapeutic methods and is relevant to the development of prophylactic and/or therapeutic treatments. It is to be appreciated that all references herein to treatment include curative, palliative and prophylactic treatment.

Prophylaxis or therapy includes but is not limited to eliciting an effective decrease in TLR4 amount, function or activity in order to cause a reduction in one or more symptoms or conditions associated with, or resulting from ACLF. The symptoms or conditions may be, for example, any of those discussed above. For example, prophylaxis or therapy may result in: reduced symptoms of renal dysfunction, prevention or reduced symptoms of liver failure, reduced levels of plasma creatinine, plasma ammonia, liver enzyme concentrations (such as reduced concentrations of ALT and/or AST in the liver), reduced inflammation in the liver and/or kidney and/or brain and/or blood circulation, and or a reduction in liver tissue damage resulting from liver failure, such as resulting from acetaminophen (APAP) toxicity. Prophylaxis or therapy may result in the maintenance of a particular level of renal dysfunction, renal failure, plasma creatinine, brain dysfunction and/or brain swelling, plasma ammonia, liver enzyme concentrations (such as concentrations of ALT and/or AST in the liver), inflammation in the liver and/or kidney and/or brain and/or blood circulation, and or liver tissue damage resulting from liver failure, such as resulting from acetaminophen (APAP) toxicity, in a patient where such symptoms have been increasing or are expected to increase as a result of the ACLF. Prophylaxis or therapy may result in such changes in symptoms or conditions in such an individual changing at a reduced rate compared to the changes that would have been seen or would have been expected in the absence of such treatment.

Prophylaxis or therapy may have similar effects in relation to any of the symptoms or consequences of liver failure such as ALF or ACLF described herein. That is, treatment in accordance with the present invention may lead to a lessening in the severity of such symptoms or consequences, maintenance of an existing level of such symptoms or consequences or a slowing or reduction in the worsening of such symptoms or consequences.

### Patients to be Treated

The present invention relates to the treatment or prevention of liver failure such as ALF or ACLF in individuals in need thereof. An individual to be treated in accordance with the present invention may therefore have liver failure such as ALF or ACLF or may be at increased risk of liver failure such as ALF or ACLF. For example, the subject may have liver failure. The subject may have immune dysfunction or failure, systemic inflammation, renal failure or brain dysfunction and/or brain swelling. The subject may have AH, NAFLD or NASH.

Methods for diagnosing liver failure, immune dysfunction, renal dysfunction, brain dysfunction, brain swelling or immune failure are well known in the art and in particular to clinicians and veterinarians in the field. For example, renal dysfunction is characterised by a reduction or loss of renal function, which may be assessed by monitoring urine volume, or sodium concentration and osmolality of the urine. Hepatorenal syndrome is also associated with a reduction in renal blood flow. Preferably, the subject will have been diagnosed as having liver failure for example by a medical or veterinarian professional. The subject may display one or more symptoms associated with liver failure, renal dysfunction or renal failure.

Methods for diagnosing liver failure such as ACLF are well known in the art and in particular to clinicians and veterinarians in the field. ACLF is diagnosed by use of the Chronic Liver Failure (CLiF) Consortium criteria, NACSELD criteria or APASL criteria. Previously validated scores to assess disease severity include Child-Pugh (CP) classification, Model for End Stage Liver Disease (MELD) and the CLiF Consortium Acute Decompensation (CLIF-C AD) score.

The individual to be treated may have increased expression of TLR4 in the liver compared with a healthy individual, such as an individual not having liver failure such as ALF or ACLF. The individual to be treated may have increased serum or plasma TLR4 compared with a healthy individual, such as an individual not having liver failure such as ALF or ACLF.

The individual to be treated may have been diagnosed as suffering from liver failure such as ALF or ACLF, or one or more symptoms or conditions as described herein that may be associated with liver failure such as ALF or ACLF, for example by any of these methods. The individual to be treated may have been diagnosed as being at risk of liver failure such as ALF or ACLF. For example, the individual may have been diagnosed with one or more symptoms that are associated with liver failure, cirrhosis, renal failure and/or renal failure. For example, the individual to be treated may have liver cirrhosis, AH, NASH, idiopathic non-cirrhotic portal hypertension, congenital hepatic fibrosis, partial nodular transformation, Budd-Chiari syndrome, portal vein thrombosis, right heart failure or schistosomiasis infection.

The subject to be treated may be any individual who is susceptible to liver failure such as ALF or ACLF. The subject may be male or female. Women may be more susceptible to the adverse effects of alcohol than men. Women can develop chronic liver disease in a shorter time frame and from smaller amounts of alcohol than men.

The subject to be treated may be a human. The subject to be treated may be a non-human animal. The subject to be treated may be a farm animal for example, a cow or bull, sheep, pig, ox, goat or horse or may be a domestic animal such as a dog or cat. The subject may or may not be an animal model for liver disease. The animal may be any age, but will often be a mature adult subject.

### Examples

### Example 1- Stem cell mobilisation has deleterious consequences in acute-on-chronic liver failure (ACLF)

In three small randomised trials from Asia stem cell mobilisation significantly increased survival, improved organ function and decreased the rate of infections thus suggesting stem cell mobilisation as a novel therapeutic option in ACLF. However, the present inventors generated data showing unexpectedly that stem cell mobilisation in the context of ACLF is deleterious.

First, they performed the first multicentre randomised controlled trial testing G-CSF in ACLF in Germany which was funded by the German research foundation (DFG) (NCT02669680). They recruited 163 patients randomised in two groups of G-CSF treatment and standard of care. G-CSF did not improve the survival rate and tend to increased mortality in subgroups of patients with alcoholic hepatitis and ACLF according to the APASL criteria (11).

Second, due to the conflicting data concerning the therapeutic benefit of G-CSF the present inventors performed pre-clinical studies in well-established rodent models of ACLF in order to clarify the effect of stem cell mobilisation. C57BL/6 Mice were gavaged with carbon tetrachloride (CCL4) for 6 weeks to induce liver fibrosis and subsequently injected with lipopolysaccharide (LPS) to induce tissue injury and organ failure. One hour after LPS injection mice were treated with G-CSF subcutaneously 250µg/kg once daily for 5 consecutive days (**Fig. 1A**). CCl4 induced significant bridging fibrosis and a severe endotoxin driven liver injury with extended TUNEL positive areas indicating apoptotic/necroptotic parenchymal cells. All animals without treatment survived 5 days follow up after LPS injection. However, G-CSF therapy led to a mortality of 50% after 48-hours, higher degree of liver fibrosis (Sirius Red) (**Fig. 1B****, C**). This was accompanied by increasing hepatic fibrosis and hepatic infiltration of macrophages (immunohistochemistry F4/80+) (**Fig. 1C**), which persisted after 5-days of G-CSF treatment.

Further repeats by the scientists led to a mortality of 66% in all ACLF animals treated with G-CSF within 48-hours after LPS injection, whereas all other animals survived the entire treatment period.

The treatment with G-CSF increased the mortality rate, aggravated liver macrophage infiltration and local inflammatory reactions (IL6) (**Fig. 4D**) and enhanced liver fibrosis.
**Therefore G-CSF aggravates endotoxin driven ACLF and ACLF related mortality, which is associated with worsening hepatic macrophage infiltration.**

### Example 2: Stem cell mobilisation and toll-like receptor 4 inhibition acts synergistically as a treatment in acute-on-chronic liver failure.

The inventors investigated whether Toll-like receptor 4 (TLR4) inhibition prevents harm from stem cell mobilising agents in acute-on-chronic liver failure, and whether an aggravated inflammatory reaction and organ dysfunction was linked to stem cell mobilisation. All stem cell mobilising agents such as G-CSF, GM-CSF, and AMD3100, share that they are effective in mobilising stem cells but simultaneously release pro-inflammatory cells such as neutrophils and monocytes. The inventors hypothesised that once released into the circulation in acute-on-chronic liver failure those cells are immediately confronted with circulating danger molecules such as damage associated molecular patterns (DAMPs) and pathogen associated molecular patters (PAMPs) which activate immune cells via the Toll-like receptor 4 (TLR4). The subsequent exaggerated inflammatory reaction aggravates tissue injury.

The present inventors hypothesised that the increased G-CSF-related mortality in ACLF may be due to a TLR4-dependent mechanism based upon the following observations:
- Sensitisation to endotoxins via TLR4 up-regulation in the liver is a key event in cirrhosis (**Fig. 2A****, B**).
- G-CSF increases TLR4 expression, as it has been shown that G-CSF increased hepatic TLR4 expression in a murine model of liver failure and aggravated liver injury once G-CSF was administered to this model (12).
- The inventors found that the number of TLR4 +ve CD45+ immune cells increased in the liver (**Fig. 2C**).

In order to explore the hypothesis, the present inventors used the TLR4 antagonist, TAK-242 in the rodent of ACLF models with or without G-CSF. TAK-242 was effective in reducing cell death in the liver (**Fig. 2D**) and improving plasma ALT levels (**Fig. 2D**).

The inventors then tested whether TLR4 inhibition (e.g. TAK-242) rescues from G-CSF driven injury and inflammation in preclinical models of ACLF. C57B/6 mice were gavaged for 6 weeks with CCl4, subsequently injected with LPS and treated with either G-CSF or TAK-242 or its combination for 24 hours and 5 days (**Fig. 3A****,** **Fig. 4A**). The results showed that the animals were protected from G-CSF-related death (**Fig. 3D**) and there was a reduction in plasma ALT levels (**Fig. 3B**), an amelioration of LPS induced organ injury evidenced by reduction in liver cell death (TUNEL (**Fig. 3B**), BCL2 (**Fig. 4E**)) and inflammatory response (macrophage infiltration, liver neutrophils, (**Fig. 3C****, D**), TNFalpha, IL6 (**Fig .4D**)) in an LPS driven inflammatory LPS model.

The STAT3 pathway mitigates against apoptosis through release of BCL2, which antagonises BAX. The inventors also observed that the protective effect of TAK-242/G-CSF was associated with activation of the STAT3 pathway (increased pSTAT3) (**Fig. 4F**), possibly induced by IL-22 secretion (**Fig4G** **Regeneration**), with increased expression of anti-apoptotic BCL2 and higher BCL2/BAX ratio (**Fig. 4F**). Likewise, injury markers (such as Lipocalin/NGAL and IL-13) were increased with G-CSF but reduced if TAK-242 was added as shown by the protein profiler after pooling of liver lysates (**Fig 4G** **Inflammation/Injury and Senescence**). LPS injection was also associated with stellate cells activation (aSMA expression) which could be significantly abrogated by TAK-242 (**Fig 4H**).

Monocytes and macrophages are major source of cytokine release in liver disease. The inventors therefore tested *in vitro,* to what extent LPS and G-CSF modulate the cytokine response in PMA activated and native THP1 cells. These experiments showed that G-CSF incubation in PMA-activated THP1 macrophage-like cells and native THP1 monocyte-like cells prior to LPS stimulation resulted in aggravated cytokine response, especially up-regulation of IL6 mRNA expression [THP1 macrophages + LPS 10ng/mL: 6.2 fold upregulation vs. LPS 10ng/mL + G-CSF 100ng/mL: 6.7 fold upregulation; THP1 monocytes + LPS 10ng/mL: 60.2 fold upregulation vs. LPS 10ng/mL + G-CSF 100ng/mL: 71 fold upregulation (**Fig 4I**)]. These *in vitro* findings are in keeping with hepatic IL6 levels, which increased 2.8-fold in G-CSF-treated animals (**Fig. 4D**). These changes either with LPS alone or in combination with G-CSF were completely prevented in the presence of TAK-242 showing clearly that TAK-242 prevents the overwhelming inflammatory response driven by LPS and G-CSF. The data help to explain the reduction in markers of organ injury in the ACLF animals treated with G-CSF/TAK-242.

The inventors observed that the combinatory therapy with TAK-242 and G-CSF has synergistic effects on liver regeneration and injury. G-CSF in combination with TAK242 reduced hepatocyte senescence after 24 hours and significantly improved hepatocyte proliferation after 5 days treatment whilst the individual treatment with TAK242 did not enhance hepatocyte driven tissue repair (**Fig. 4B****, C, D**). In CCL4 cirrhotic animals that were not administered LPS, the present inventors observed similar G-CSF effect on hepatocyte proliferation thereby providing the evidence that prevention of LPS-TLR4 signalling is the key to unmasking the pro-proliferative ability of G-CSF.

As a further proof that G-CSF exhibits a pro-proliferative effect in a non-inflammatory environment, the inventors tested 5-days of G-CSF therapy after CCl4 administration without LPS. In this setting, G-CSF injection significantly increased the number of proliferating hepatocytes; Ki67 staining was observed in 4.1% ± 2.9 (p<0.001 to CCl4 + LPS + G-CSF 5d) and Cyclin A2 in 2.1% ± 1.3 (p<0.05 to CCl4 + LPS + G-CSF 5d) (**Fig 4J**).

Protein expression of other regenerative markers were assessed in pooled liver lysates by using the proteome profiler. TAK-242 alone, administered over 24-hours in the CCl4-LPS mice, reduced both hepatic markers of vascular regeneration (such as angiopoietin 2, proliferin and PDGF) and other markers involved in liver regeneration, such as IL-22, Flt3-ligand and IGFBP-1 (**Fig4G** **Vascular regeneration and Regeneration**). Adding G-CSF to TAK-242 markedly increased the liver protein expression of these pro-regenerative markers (**Fig4G** **Vascular regeneration and Regeneration**).

These findings confirmed the hypothesis that G-CSF requires a non-inflammatory environment to exert its proliferative effects on hepatocytes and, therefore, anti-inflammatory therapy with TAK-242 allows G-CSF to unfold its pro-regenerative capacities.

### Example 3: Mechanism of the synergy between G-CSF and TAK-242 in a non-inflammatory ACLF model

It is well known that G-CSF has pro-proliferative properties and cirrhosis is associated with cellular senescence and a lack of hepatocyte proliferation. The present inventors therefore evaluated whether the mechanism of the beneficial effect of TAK-242 and G-CSF as combined therapy is due to modulation of inflammation whilst increasing hepatocyte proliferation.

It is well described from several pre-clinical models of liver injury that stem cell mobilisation and homing into the liver induces parenchymal regeneration. However, ACLF is a distinct disease entity as it primarily links a sepsis like systemic inflammatory reaction with tissue injury, in contrast to models with direct hepatotoxic injury. Inflammation might mask regeneration induced by stem cells and regulatory immune cells. The inventors therefore tested, whether adding TAK-242 to G-CSF not only prevents G-CSF driven inflammatory response but also allows stem cell mobilisation to unfold its tissue repair capacities. The combinatory treatment of G-CSF and TAK-242 was tested in a distinct models of ACLF consisting of 6 weeks gavaging with CCL4 followed by injection of Galactosamin (hepatotoxic, non-inflammatory hit) (**Fig. 5**). In the GalN model, TAK-242 + G-CSF was superior over the individual therapies in reducing tissue injury (TUNEL (**Fig**. **5B**), BCL2 (**Fig**. **7**), ALT levels (**Fig**. **5B**) and RIPK3 (**Fig**. **6A**, **B**)).

In the GalN induced non-inflammatory model adding TAK-242 to G-CSF was significantly superior to the individual therapies in reducing liver injury (TUNEL, RIPK3) via BCL2 up-regulation (**Fig**. **7**).

In the GalN induced non-inflammatory model GalN injection induced a significant liver injury with high ALT levels [27.3 U/L (range 24.2-63.4) vs. 288 U/L (range 46-807) (p<0.001)], extended areas of cell death (TUNEL) (0.23% (range 0.17-0.29) vs. 2.6% (range 1.3-5.2), and RIPK3 positivity [1% (range 0.75-1.1) vs. 3.2% (range 1.6-4.4), p<0.001)], a marker for necroptotic cell death. In contrast to the inflammatory LPS model, treatment with G-CSF alone reduced ALT levels [101 U/L (range 51-284)], liver cell death (TUNEL 1.2% (range 0.4-1.9), p<0.001) and RIPK3 expression [1.9% (range 0.9-2.5)]. TAK-242 alone tended to reduce ALT levels to 96.5 U/L (range 45-229), RIPK3 expression [1.7% (range 0.9-2.1)] and liver cell death [TUNEL 1.4% (range 0.4-3.3)], however without reaching statistical significance. The combination of G-CSF and TAK242 was superior to both single treatments and improved ALT levels; 74.5 U/L (range 44-297), liver cell death (TUNEL 0.45% (0.11-0.95), p<0.001; compared with both individual therapies) and RIPK3 expression (1.23% (1-1.78), p<0.01 to CCl4+GalN). Furthermore, treatment with TAK-242 + G-CSF was also associated with activation of the STAT3 pathway and a trend to increased anti-apoptotic BCL2 expression as assessed in pooled liver samples by Western Blot.

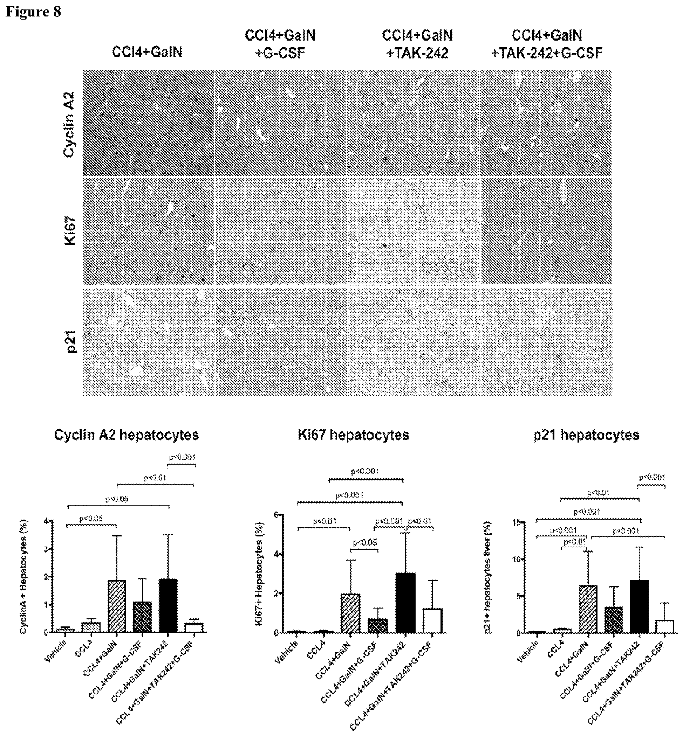

The inventors also evaluated whether hepatocyte proliferation was modulated by treatment with G-CSF ± TAK-242 in this model. CCl4-treated animals injected with GalN showed high levels of proliferating hepatocytes [Ki67: CCl4 0.1% ± 0.1 vs. CCl4 + GalN 2% ± 1.7, (p<0.01), Cyclin A2: CCl4 0.1% ± 0.2 vs. CCl4 + GalN 1.9% ± 1.6, (p<0.05)] and hepatocytes in cell cycle arrest (p21: CCl4 0.5% ± 0.2 vs. CCl4 + GalN 6.4% ± 4.7, p<0.01). Treatment with G-CSF, with or without TAK-242, reduced the degree of proliferating and senescent hepatocytes, whereas TAK-242 alone did not alter the response to injury [(Ki67: CCl4 + GalN + TAK-242 - 3% ± 2.1 vs. CCl4 + GalN + TAK-242 + G-CSF: 1.2% ± 1.4 (p<0.05); Cyclin A2: CCl4 + GalN + TAK-242 - 1.9% ± 1.6 vs. CCl4 + GalN + TAK-242 + G-CSF 0.3% ± 0.2 (p<0.001); p21: CCl4 + GalN + TAK-242 7.1% ± 4.5 vs. CCl4 + GalN + TAK-242 + G-CSF 1.7% ± 2.3 (p<0.001)] (**Fig. 8**).

Taken together these data suggest that in this non-inflammatory model, the combination of G-CSF and TAK-242 impacts positively on liver injury, regeneration and also reduces markers of senescence. It also confirms that the regenerative effect of G-CSF is preserved in a non-inflammatory environment.

### Example 4 - Relationship between cell death and liver regeneration and the effect of G-CSF and TAK-242

In order to explore the role of cell death as a modulator of regeneration, the inventors used RIPA56, a selective RIPK1 inhibitor to prevent GalN-induced necroptotic cell death in the different treatment groups. RIPA56 treatment effectively prevented GalN-driven cell death (TUNEL p<0.001 and RIPK3 p<0.001 both compared to CCl4 + GalN) associated with a decrease in hepatocyte proliferation compared to CCl4 + GalN (Ki67 2% ± 1.7 vs. 0.2% ± 0.2, p<0.001; Cyclin A2 1.9% ± 1.6 vs. 0.03% ± 0.1, p<0.001) (**Fig. 9**), which suggests that in this non-inflammatory environment, the regenerative response correlates directly with the degree of injury.

The inventors then calculated the ratio between expression of Cyclin A2, as a marker of cell cycle progression, and the amount of liver cell death (TUNEL) to delineate the pro-regenerative capacity in relation to liver injury of both drugs in all models studied. An increase in the ratio between Cyclin A2 and TUNEL therefore depicts enhanced regenerative activity with less cell death and vice versa (**Fig. 10**). In the 24-hour CCl4-LPS model, LPS injection completely inhibited regenerative responses (CyclinA2/TUNEL ratio: CCl4 0.42 vs. CCl4 + LPS 0.02 vs. CCl4 + LPS + G-CSF 0.04), with or without G-CSF; in contrast, in the CCl4-GalN model the regenerative response was preserved after GalN injection (CyclinA2/TUNEL ratio: CCl4 + GalN 0.54). In both "short-term" models, administration of TAK-242 was associated with enhanced liver cell regenerative response (CyclinA2/TUNEL ratio: CCl4 + LPS + TAK-242: 1.4; CCl4 + GalN + TAK-242: 0.9) supporting the hypothesis that creating an inflammation-free environment is important to restore regenerative capacities. Moreover, the anti-inflammatory environment (in presence of TAK-242) allowed G-CSF to unfold its positive effect on liver injury, notably in the CCl4-LPS model, and to enhance regeneration after 5-days of therapy (CyclinA2/TUNEL ratio: CCl4 + LPS + TAK-242 + G-CSF: 1.7), thus supporting the pro-regenerative capacity of G-CSF especially after long-term treatment.

### Conclusions

The data that shows a synergy between G-CSF and TLR4 inhibition using TAK-242 by impacting on hepatic proliferation whilst modulating the severity of inflammation, thereby improving survival of models of ACLF. G-CSF is an approved treatment for neutropenia after chemotherapy and for bone marrow stimulation in healthy stem cell donors. G-CSF has a long-term proven safety record and can be repurposed. It is available for clinical use and is a TRL9. TAK-242, the TLR4 antagonist has been tested in clinical trials up to a Phase 3 and has a proven safety record. TAK-242 recently acquired status TRL8 and is about to be tested in a phase 2a/2b clinical trial.

The inventors therefore have shown the following unexpected findings:
(i) In clinically relevant animal models, the stem cell mobiliser, G-CSF is deleterious as it induces systemic inflammation and increases death rates
(ii) TAK-242 inhibits inflammation and organ injury and (ii) reduces liver inflammation thereby allowing hepatic stem cells to engraft;
(iii) G-CSF mobilises stem cells but these cells cannot engraft in the liver due to hepatic inflammation and G-CSF can lead to worsened inflammation and increases death rates in animal models;
(iv) The combination of G-CSF, the stem cell mobiliser, and TAK242 acts synergistically to (i) reduce liver and systemic inflammation (ii) mobilise stem cells without the inducing inflammation (iii) these cells have an improved ability to engraft in the liver.

### References

1. Moreau R, Jalan R, Gines P, Pavesi M, Angeli P, Cordoba J, Durand F, et al. Acute-on-chronic liver failure is a distinct syndrome that develops in patients with acute decompensation of cirrhosis. Gastroenterology 2013;144:1426-1437, 1437 e1421-1429.
2. Jalan R, Saliba F, Pavesi M, Amoros A, Moreau R, Gines P, Levesque E, et al. Development and validation of a prognostic score to predict mortality in patients with acute-on-chronic liver failure. J Hepatol 2014;61:1038-1047.
3. Albillos A, Lario M, Alvarez-Mon M. Cirrhosis-associated immune dysfunction: distinctive features and clinical relevance. J Hepatol 2014;61:1385-1396.
4. Engelmann C, Sheikh M, Sharma S, Kondo T, Loeffler-Wirth H, Zheng YB, Novelli S, et al. Toll-like receptor 4 is a therapeutic target for prevention and treatment of liver failure. J Hepatol 2020.
5. am Esch JS, Schmelzle M, Furst G, Robson SC, Krieg A, Duhme C, Tustas RY, et al. Infusion of CD133+ bone marrow-derived stem cells after selective portal vein embolization enhances functional hepatic reserves after extended right hepatectomy: a retrospective single-center study. Ann Surg 2012;255:79-85.
6. Garg V, Garg H, Khan A, Trehanpati N, Kumar A, Sharma BC, Sakhuja P, et al. Granulocyte colony-stimulating factor mobilizes CD34(+) cells and improves survival of patients with acute-on-chronic liver failure. Gastroenterology 2012;142:505-512 e501.
7. Duan XZ, Liu FF, Tong JJ, Yang HZ, Chen J, Liu XY, Mao YL, et al. Granulocyte-colony stimulating factor therapy improves survival in patients with hepatitis B virus-associated acute-on-chronic liver failure. World J Gastroenterol 2013;19:1104-1110.
8. Saha BK, Mahtab MA, Akbar SMF, Noor EASM, Mamun AA, Hossain SMS, Alam MA, et al. Therapeutic implications of granulocyte colony stimulating factor in patients with acute-on-chronic liver failure: increased survival and containment of liver damage. Hepatol Int 2017;11:540-546.
9. Spahr L, Lambert JF, Rubbia-Brandt L, Chalandon Y, Frossard JL, Giostra E, Hadengue A. Granulocyte-colony stimulating factor induces proliferation of hepatic progenitors in alcoholic steatohepatitis: a randomized trial. Hepatology 2008;48:221-229.
10. Newsome PN, Fox R, King AL, Barton D, Than NN, Moore J, Corbett C, et al. Granulocyte colony-stimulating factor and autologous CD133-positive stem-cell therapy in liver cirrhosis (REALISTIC): an open-label, randomised, controlled phase 2 trial. Lancet Gastroenterol Hepatol 2018;3:25-36.
11. Engelmann C, Herber A, Bruns T, Schiefke I, Zipprich A, Schmiedeknecht A, Zeuzem A, et al. Granulocyte-colony stimulating factor (G-CSF) to treat acute-on-chronic liver failure (GRAFT trial): Interim analysis of the first randmised European trial.. Hepatology 2019;70:1476.
12. Fang H, Liu A, Sun J, Kitz A, Dirsch O, Dahmen U. Granulocyte colony stimulating factor induces lipopolysaccharide (LPS) sensitization via upregulation of LPS binding protein in rat. PLoS One 2013;8:e56654.

Sugiyama et al (European Journal of Pharmacology 594 (2008) 152-156).
Hennessy et al (2010) Nature Reviews Drug Discovery 9: 293-307.
Harlow and Lane (1988) "Antibodies: A Laboratory Manual", Cold Spring Harbor Laboratory Press, Cold Spring Harbor, NY.
Maddox et al, J. Exp. Med. 158, 1211-1226, 1993.

## Claims

1. A combination of:
(a) a stem cell mobiliser selected from granulocyte colony stimulating factor (G-CSF), filgrastim, lenograstim and pegfilgrastim; and
(b) an antagonist of TLR4,
for use in a method of treating or preventing liver failure in an individual in need thereof.

2. The combination for use according to claim 1, wherein the liver failure is acute liver failure (ALF) or acute-on-chronic liver failure (ACLF).

3. The combination for use according to claim 2, wherein the individual is suffering from, or is at risk of one or more of the following, when compared to a subject not suffering from ACLF:
(a) renal dysfunction, and/or
(b) renal failure, and/or
(c) brain dysfunction, and/or
(d) brain swelling, and/or
(e) inflammation, injury or dysfunction in the kidney and/or brain, and/or
(f) liver failure, and/or
(g) immune failure.

4. The combination for use according to any one of the preceding claims, wherein the stem cell mobiliser and/or the antagonist of TLR4 is administered in combination with a further agent useful in the treatment or prevention of liver failure, such as ALF or ACLF.

5. The combination for use according to any one of the preceding claims, wherein the stem cell mobiliser is G-CSF.

6. A stem cell mobiliser selected from granulocyte colony stimulating factor (G-CSF), filgrastim, lenograstim, and pegfilgrastim for use in a method of treating or preventing liver failure in an individual in need thereof, wherein the method involves the additional administration of an antagonist of TLR4 to the individual.

7. The stem cell mobiliser for use according to claim 6, wherein the administration of the antagonist of TLR4 precedes the administration of the stem cell mobiliser to the individual, is at the same time as the administration of the stem cell mobiliser to the individual, is sequential to the administration of stem cell mobiliser to the individual, or is subsequent to the administration of the stem cell mobiliser to the individual.

8. The stem cell mobiliser for use according to claim 6 or 7, wherein the stem cell mobiliser is G-CSF.

9. The stem cell mobiliser and an antagonist of TLR4 for use according to any one of claims 1 to 5, or the stem cell mobiliser for use according to any one of claims 6 to 8, wherein the antagonist of TLR4 is TAK-242.

## Patentansprüche

1. Kombination aus:
(a) einem Stammzellmobilisierer, ausgewählt aus Granulozyten-Koloniestimulierungsfaktor (G-CSF), Filgrastim, Lenograstim und Pegfilgrastim; und
(b) einem Antagonisten von TLR4,
für die Verwendung in einem Verfahren zum Behandeln und Vorbeugen von Leberversagen bei einem Individuum, das dessen bedarf.

2. Kombination für die Verwendung nach Anspruch 1, wobei das Leberversagen akutes Leberversagen (ALF) oder akut-auf-chronisches Leberversagen (ACLF) ist.

3. Kombination für die Verwendung nach Anspruch 2, wobei das Individuum im Vergleich zu einem Subjekt, das nicht an ACLF leidet, an einem oder mehreren der folgenden leidet oder einem Risiko dafür ausgesetzt ist:
(a) Nierenfunktionsstörung und/oder
(b) Nierenversagen und/oder
(c) Hirnfunktionsstörung und/oder
(d) Hirnschwellung und/oder
(e) Entzündung, Verletzung oder Funktionsstörung in der Niere und/oder im Gehirn und/oder
(f) Leberversagen und/oder
(g) Immunversagen.

4. Kombination für die Verwendung nach einem der vorhergehenden Ansprüche, wobei der Stammzellmobilisierer und/oder der Antagonist von TLR4 in Kombination mit einem weiteren Mittel verabreicht wird, das bei der Behandlung oder Vorbeugung von Leberversagen, wie ALF oder ACLF, nützlich ist.

5. Kombination für die Verwendung nach einem der vorhergehenden Ansprüche, wobei der Stammzellmobilisierer G-CSF ist.

6. Stammzellmobilisierer, ausgewählt aus Granulozyten-Koloniestimulierungsfaktor (G-CSF), Filgrastim, Lenograstim und Pegfilgrastim, für die Verwendung in einem Verfahren zum Behandeln oder Vorbeugen von Leberversagen bei einem Individuum, das dessen bedarf, wobei das Verfahren die zusätzliche Verabreichung eines Antagonisten von TLR4 an das Individuum einschließt.

7. Stammzellmobilisierer für die Verwendung nach Anspruch 6, wobei die Verabreichung des Antagonisten von TLR4 der Verabreichung des Stammzellmobilisierers an das Individuum vorhergeht, gleichzeitig mit der Verabreichung des Stammzellmobilisierers an das Individuum erfolgt, auf die Verabreichung des Stammzellmobilisierers an das Individuum folgt oder nach der Verabreichung des Stammzellmobilisierers an das Individuum erfolgt.

8. Stammzellmobilisierer für die Verwendung nach Anspruch 6 oder 7, wobei der Stammzellmobilisierer G-CSF ist.

9. Stammzellmobilisierer und Antagonist von TLR4 für die Verwendung nach einem der Ansprüche 1 bis 5 oder Stammzellmobilisierer für die Verwendung nach einem der Ansprüche 6 bis 8, wobei der Antagonist von TLR4 TAK-242 ist.

## Revendications

1. Combinaison de :
(a) un mobilisateur de cellules souches sélectionné parmi le facteur de stimulation de colonies de granulocytes (G-CSF), le filgrastim, le lénograstim et le pegfilgrastim ; et
(b) un antagoniste de TLR4,
destinée à être utilisée dans un procédé de traitement ou de prévention de l'insuffisance hépatique chez un individu qui en a besoin.

2. Combinaison destinée à être utilisée selon la revendication 1, dans laquelle l'insuffisance hépatique est une insuffisance hépatique aiguë (ALF) ou une insuffisance hépatique aiguë sur chronique (ACLF).

3. Combinaison destinée à être utilisée selon la revendication 2, dans laquelle l'individu souffre ou est à risque d'un ou plusieurs des troubles suivants, par rapport à un sujet ne souffrant pas d'ACLF :
(a) un dysfonctionnement rénal, et/ou
(b) une insuffisance rénale, et/ou
(c) un dysfonctionnement cérébral, et/ou
(d) un œdème cérébral, et/ou
(e) une inflammation, une lésion ou un dysfonctionnement des reins et/ou du cerveau, et/ou
(f) une insuffisance hépatique, et/ou
(g) un dysfonctionnement immunitaire.

4. Combinaison destinée à être utilisée selon l'une quelconque des revendications précédentes, dans laquelle le mobilisateur de cellules souches et/ou l'antagoniste de TLR4 est administré en combinaison avec un autre agent utile dans le traitement ou la prévention de l'insuffisance hépatique, telle que l'ALF ou l'ACLF.

5. Combinaison destinée à être utilisée selon l'une quelconque des revendications précédentes, dans laquelle le mobilisateur de cellules souches est G-CSF.

6. Mobilisateur de cellules souches sélectionné parmi le facteur de stimulation des colonies de granulocytes (G-CSF), le filgrastim, le lénograstim et le pegfilgrastim pour une utilisation dans un procédé de traitement ou de prévention de l'insuffisance hépatique chez un individu qui en a besoin, dans lequel le procédé implique l'administration supplémentaire d'un antagoniste de TLR4 à l'individu.

7. Mobilisateur de cellules souches destiné à être utilisé selon la revendication 6, dans lequel l'administration de l'antagoniste de TLR4 précède l'administration du mobilisateur de cellules souches à l'individu, se fait en même temps que l'administration du mobilisateur de cellules souches à l'individu, est séquentielle à l'administration de mobilisateur de cellules souches à l'individu, ou est postérieure à l'administration du mobilisateur de cellules souches à l'individu.

8. Mobilisateur de cellules souches destiné à être utilisé selon la revendication 6 ou 7, dans lequel le mobilisateur de cellules souches est G-CSF.

9. Mobilisateur de cellules souches et antagoniste de TLR4 destinés à être utilisés selon l'une quelconque des revendications 1 à 5, ou mobilisateur de cellules souches destiné à être utilisé selon l'une quelconque des revendications 6 à 8, dans lequel l'antagoniste de TLR4 est TAK-242.
